(19) Europäisches Patentamt
European Patent Office
Office européen des brevets

(11) EP 3 187 172 A1

(12) EUROPEAN PATENT APPLICATION

(43) Date of publication:
**05.07.2017 Bulletin 2017/27**

(51) Int Cl.:
***A61K 9/107*** *(2006.01)* ***A61K 38/13*** *(2006.01)*

(21) Application number: **16382001.2**

(22) Date of filing: **04.01.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicants:
- **Spherium Biomed S.L.**
  **08950 Esplugues de Llobregat, Barcelona (ES)**
- **Bionanoplus, S.L.**
  **31110 Noáin- Navarra (ES)**

(72) Inventors:
- **SALMAN, Hesham H.A.**
  **E-31110 Noáin-Navarra (ES)**
- **ESPARZA CATALÁN, Irene**
  **E-31110 Noáin-Navarra (ES)**
- **SANTOS LOBO, Benjamín**
  **E-08950 Esplugues de Llobregat, Barcelona (ES)**
- **LLUCH LAFUENTE, Nuria**
  **E-08950 Esplugues de Llobregat, Barcelona (ES)**

(74) Representative: **ABG Patentes, S.L.**
**Avenida de Burgos, 16D**
**Edificio Euromor**
**28036 Madrid (ES)**

# (54) CYCLOSPORINE A TOPICAL COMPOSITIONS

(57) The present invention relates to topical pharmaceutical microemulsions of cyclosporine A comprising a half ($C_1$-$C_4$) alkyl esters of poly (methyl vinyl ether-co-maleic anhydride) (PVM/MA) copolymers. The invention also relates to a process for the preparation of said compositions and to their use in the prevention and/or treatment of several diseases, particularly psoriasis and atopic dermatitis.

EP 3 187 172 A1

## Description

## FIELD OF THE INVENTION

The invention relates to pharmaceutical compositions of cyclosporine A for topical application, to a process for the production of said compositions and to their use in medicine, particularly in the prevention and/or treatment of psoriasis or atopic dermatitis.

## BACKGROUND OF THE INVENTION

Cyclosporine A was discovered in 1972 while searching for novel antifungal agents. It is a cyclic polypeptide consisting of 11 amino acids and is produced as a metabolite by the fungus species *Beauveria nivea.* Although cyclosporine A was initially noted to have only a narrow antifungal spectrum, it was subsequently found to be a potent immunosuppressive drug in 1976.

Cyclosporine A was the first immunosuppressive drug found to act selectively on T cells. The helper T cell is the main target, but the T suppressor cell may also be affected. Cyclosporine A forms a complex with cyclophilin, an intracellular immunophilin, and inhibits the activity of calcineurin phosphatase, a calcium/calmodulin-dependent serine-threonine phosphatase. As a result, calcineurin phosphatase is unable to phosphorylate nuclear factor of activated T cells (NFAT), a transcription factor. NFAT requires phosphorylation before transportation to the nucleus for transcription of genes encoding interleukin-2 (IL-2), a cytokine that is necessary for full activation of the T-cell pathway, interferon-gamma, and granulocyte-macrophage colony-stimulating factor (GM-CSF). Cyclosporine A depletes lymphocytes and macrophages in the epidermis and dermis and inhibits the activation of T cells, natural killer cells, and antigen-presenting cells. Cyclosporine A also inhibits keratinocyte hyperproliferation, inhibits the release of histamine from mast cells, and downregulates the expression of cellular adhesion molecules on dermal capillary endothelium [Amor et al., J. Am. Acad. Dermatol., 2010, 63, 925-946].

In 1978, cyclosporine A was found to be successful in preventing rejection in renal transplant patients who received mismatched cadaver kidneys. In 1979 it was observed that cyclosporine A improved psoriasis. In fact, cyclosporine A was approved by the FDA for the treatment of this disease in 1997. More recent reports, have disclosed that cyclosporine A is useful for the treatment of a variety of dermatological diseases. In this regard, Amor et al. [J. Am. Acad. Dermatol., 2010, 63, 925-946] disclosed that cyclosporine A successfully treated atopic dermatitis, pyoderma gangrenosum and refractory chronic idiopathic urticaria, dyshidrotic eczema, Behçet disease, pityriasis rubra pilaris, dermatomyositis, pemphigus vulgaris, epidermolysis bullosa acquisita, photodermatoses (such as chronic actinic dermatitis, polymorphic light eruption and solar urticaria),lichen planus, prurigo nodularis, alopecia areata, benign familiar pemphigus, eosinophilic pustular folliculitis, hidradenitis suppurativa and scleroderma. Cyclosporine A has also been used in veterinary dermatology; in particular, it has been reported [Kovalik et al., The Veterinary Journal, 2012, 193, 317-325] to be useful for the treatment of atopic dermatitis, sebaceous adenitis, pemphigus foliaceus and erythematosus, vesicular cutaneous lupus erythematosus, and cutaneous reactive histiocytosis, in cats and dogs. Further dermatologic indications of cyclosporine A in veterinary have been reported by Palmeiro [Vet. Clin. Small Anim., 2013, 43, 154-171], namely allergic dermatitis, eosinophilic granuloma complex, atopic dermatitis, perianal fistulas, sebaceous adenitis, pemphigus foliaceus and erythematosus, juvenile cellulitis, vesicular cutaneous lupus erythematosus, erythema multiforme, discoid lupus erythematosus, sterile nodular panniculitis, metatarsal fistulae, granulomatous folliculitis and furunculosis, nasal arteritis, ulcerative dermatosis of nasal philtrum, facial dermatitis, sterile granuloma or pyogranuloma syndrome, mular folliculitis, alopecia areata, psudopelade, cutaneous reactive hitiocytosis, feline plasma cell pododermatitis, vasculitis and ischemic dermatopathy, in cats and dogs. Cyclosporine A can also be used for the treatment of vitiligo due to its immunosuppressant activity, in particular due to its activity as calcineurin inhibitor, a class of drugs which have shown promising results in repigmentation of affected areas in patients with vitiligo [Caridi et al., Topical Calcineurin Inhibitors in the Treatment of Vitiligo in Bitiligo - Management and Therapy, Dr. Kelly KyunHwa Park Ed., 2001].

Cyclosporine A exhibits very poor solubility in water, and, as a consequence, suspension and emulsion forms were been developed for oral administration and for injection, such as Sandimmune® and Neoral® (Novartis Pharmaceuticals). However, systemic therapies, such as oral and intravenous administrations, have the drawback of producing significant side effects.

Thus, it is desirable to have topical cyclosporine A formulations suitable for topical administration of cyclosporine A, in particular to permit site specific delivery to the skin. The key challenges for topical delivery of this drug are its poor water solubility, high molecular weight and limited cutaneous permeation.

Ophthalmic emulsions containing cyclosporine A, glycerin, castor oil, polysorbate 80, carbomer copolymer A, and water, have been disclosed [US 8,642,556 B2; US 8,629,111 B2; US 8,618,064 B2; US 8,633,162 B2; US 8,648,048 B2; and US 8,685,930 B2], one of them being marketed under the trademark Restasis®. However, the concentration of cyclosporine A in these emulsions is not greater than 0.1% by weight.

Topical compositions comprising higher concentrations of cyclosporine A, from 0.1 to 10% by weight, can be formulated by using a polyalkyl ester of polycarboxylic acid, such as adipic, pimelic, azelaic, sebacic and phthalic dialkyl esters, as disclosed in US 5,891,846. However, manufacturing these formulations requires the use of complex equipment and/or processes.

Therefore, there is a need in the art to develop further topical compositions comprising cyclosporine A, in particular that allow the local delivery of cyclosporine A to skin compartment, capable of containing high concentrations of cyclosporine A, having high stability. It is also desirable that the preparation of these compositions avoid the use of toxic organic solvents or complex techniques.

## SUMMARY OF THE INVENTION

The inventors have surprisingly found that microemulsions comprising a half $C_{1-4}$-alkylester of a poly(methyl vinyl ether-co-maleic anhydride) (PVM/MA) copolymer, a medium chain triglyceride, a volatile alcohol, 2-(2-ethoxyethoxy)ethanol, a non-volatile organic solvent capable of solubilizing the copolymer, and a surfactant or mixture of surfactants having an HLB value from 10 to 18, are capable of containing high concentrations of cyclosporine A. These microemulsions are stable and suitable for topical application.

The microemulsions of the invention have demonstrated to have several advantages over other formulations of the prior art such as improved drug solubilization, skin bioadhesive properties, physical and chemical stability, percutaneous absorption, spontaneous formation, ease of manufacturing and scale-up and suitability for preparing formulations suitable for spraying. Said microemulsions are capable of solving all or some of the drawbacks related to other compositions of cyclosporine A, for example, low long-term stability, low encapsulation efficacy, poor drug solubilization, low percutaneous absorption, systemic absorption, a cost and complex production process which requires the use of toxic organic solvents or complex techniques.

The examples of the present invention shown that the microemulsions of the invention have high stability under accelerated storage conditions both in terms of physical stability (in particular for keeping microemulsion properties) and chemical stability (in particular avoiding degradation of cyclosporine A), and allow the local delivery of cyclosporine A to skin compartment.

In a first aspect, the invention relates to a topical pharmaceutical microemulsion comprising:

(a) from 0.1% w/w to 10%w/w of cyclosporine A relative to the total weight of the microemulsion,
(b) a half $C_{1-4}$-alkylester of a poly(methyl vinyl ether-co-maleic anhydride) (PVM/MA) copolymer,
(c) a non-volatile organic solvent capable of solubilizing component (b)other than 2-(2-ethoxyethoxy)ethanol and surfactants having an HLB value from 10 to 18,
(d) a volatile alcohol,
(e) 2-(2-ethoxyethoxy)ethanol,
(f) a medium chain triglyceride,
(g) a surfactant or surfactant mixture having an HLB value from 10 to 18,
(h) optionally water, and
(i) optionally triacetin.

In a second aspect, the invention relates to a process for producing a topical pharmaceutical microemulsion as defined in the first aspect comprising:

(i) preparing an homogeneous mixture comprising cyclosporine A and the medium chain triglyceride,
(ii) preparing an homogeneous solution comprising the half $C_{1-4}$-alkyl ester of a poly(methyl vinyl ether-co-maleic anhydride) (PVM/MA) copolymer, the volatile alcohol and the non-volatile organic solvent other than 2-(2-ethoxyethoxy)ethanol and surfactants having an HLB value from 10 to 18;
(iii) adding the solution obtained in step (ii) to the mixture obtained in step (i) under stirring,
(iv) adding 2-(2-ethoxyethoxy)ethanol, the surfactant or surfactant mixture having an HLB value from 10 to 18, and optionally triacetin to the mixture obtained in step (iii) and stirring until a homogeneous solution is obtained, and
(v) optionally adding water to the mixture obtained in step (iv) and stirring until a homogeneous solution is obtained.

In a third aspect, the invention relates to a topical pharmaceutical microemulsion as defined in the first aspect for use as a medicament, in particular a medicament for human or veterinary use.

In a fourth aspect, the invention relates to a topical pharmaceutical microemulsion as defined in the first aspect for use in the prevention and/or treatment of a disease selected from the group consisting of psoriasis, atopic dermatitis, allergic dermatitis, pyoderma gangrenosum, refractory chronic idiopathic urticaria, dyshidrotic eczema, Behçet disease, pityriasis rubra pilaris, dermatomyositis, pemphigus vulgaris, benign familiar pemphigus, pemphigus foliaceus and erythematosus, epidermolysis bullosa acquisita, photodermatoses, lichen planus, prurigonodularis, alopecia areata, eosinophilic pustular folliculitis, granulomatous folliculitis and furunculosis, mular folliculitis, hidradenitis suppurativa, scleroderma, vitiligo, eosinophilic granuloma complex, perianal fistulas, sebaceous adenitis, juvenile cellulitis, vesicular cutaneous lupus erythematosus, erythema multiforme, discoid lupus erythematosus, sterile nodular panniculitis, metatarsal fistulae, nasal arteritis, ulcerative dermatosis of nasal philtrum, facial dermatitis, sterile granuloma or pyogranuloma syndrome, pseudopelade, cutaneous reactive histiocytosis, feline plasma cell pododermatitis, vasculitis and ischemic dermatopathy.

## DETAILED DESCRIPTION OF THE INVENTION

Pharmaceutical compositions

In a first aspect, the invention relates to a topical pharmaceutical microemulsion comprising:

(a) from 0.1%w/w to 10% w/w of cyclosporine A relative to the total weight of the microemulsion,
(b) a half $C_{1-4}$-alkylester of a poly(methyl vinyl ether-co-maleic anhydride) (PVM/MA) copolymer,
(c) a non-volatile organic solvent capable of solubilizing component (b)other than 2-(2-ethoxyethoxy)ethanol and surfactants having an HLB value from 10 to 18,
(d) a volatile alcohol,
(e) 2-(2-ethoxyethoxy)ethanol,
(f) a medium chain triglyceride,
(g) a surfactant or surfactant mixture having an HLB value from 10 to 18,
(h) optionally water, and
(i) optionally triacetin.

The presence of the copolymer (component (b))in the microemulsion increases the stability of the microemulsion and provides adhesive properties to the product, therefore enhancing its adhesion to the skin.
The compositions of the invention are suitable for topical application. The term "topical" is used herein to designate application in the exterior of the body such as, without limitation, the skin, scalp and nails; and also the application to mucosae such as, without limitation, buccal, nasal or rectal mucosae.
In the context of the present invention, the terms "pharmaceutical composition" and "pharmaceutical microemulsion" are interchangeable and refer to a isotropic, thermodynamically stable transparent system made of droplets (i.e. the disperse phase), generally spherical droplets, this disperse phase being surrounded by the copolymer and with an average diameter of the disperse phase between 1 nm and 200 nm, preferably between 10 nm and 100 nm. In the context of the present invention, based on the macroscopical examination only formulations with a clear appearance (transparent formulations) were considered to be within the microemulsion range.
The term "pharmaceutical", as used herein, means that the microemulsions of the invention are pharmaceutically acceptable to the patient (such as a mammal) from a pharmacological/toxicological point of view and to the manufacturing pharmaceutical chemist from a physical/chemical point of view, due to the identity of the components forming the microemulsion.
The term "average diameter" or "mean diameter", as used herein, relates to the average diameter of a population of droplets forming the disperse phase. The average size of these systems can be measured by standard processes known by persons skilled in the art such as Dynamic light scattering (DLS or photon correlation spectroscopy, PCS), that has been described as an appropriate method for measuring droplet size in microemulsions [Goddereris C. et al., International Journal of Pharmaceutics, 2006, 312, 187-195] and is well known to the skilled person. As explained above, in the microemulsions of the present invention the average diameter of the droplets is from 1 nm to 200 nm.
The disperse phase of the microemulsions of the present invention contains cyclosporine A, the medium-chain triglyceride and optionally the non-volatile organic solvent other than 2-(2-ethoxyethoxy)ethanol and surfactants having an HLB value from 10 to 18, said disperse phase is surrounded by the PVM/MA copolymer. The non-volatile organic solvent other than 2-(2-ethoxyethoxy)ethanol and surfactants having an HLB value from 10 to 18, component (c), may be located in the disperse phase, in the continuous phase or in both phases. The polymer is located in the interphase between the disperse phase and the continuous phase of the microemulsion. The continuous phase of the microemulsions of the present invention contains the remaining components. The term "w/w", in the context of the present invention, relates to the weight of each component relative to the total weight of the microemulsion, unless otherwise stated.
Component a) in the pharmaceutical microemulsions of the present invention is cyclosporine A.
The term "cyclosporine A", as used herein, refers to a cyclic undecapeptide compound with chemical name (3*S*,6*S*,9*S*,12*R*,15*S*,18*S*,21*S*,24*S*,30*S*,33*S*)-30-ethyl-33-[(1*R*,2*R*,4*E*)-1-hydroxy-2-methyl-4-hexen-1-yl]-6,9,18,24-tetraisobutyl-3,21-diisopropyl-1,4,7,10,12,15,19,25,28-nonamethyl-1,4,7,10,13,16,19,22,25,28,31-undecaazacyclotritriacontane-2,5,8,11,14,17,20,23,26,29,32-undecone or cyclo[[(*E*)-(2*S*,3*R*,4*R*)-3-hydroxy-4-methyl-2-(methylamino)-6-octenoyl]-L-2-aminobutyryl-*N*-methylglycyl-*N*-methyl-L-leucyl-L-valyl-*N*-methyl-L-leucyl-L-alanyl-D-alanyl-*N*-methyl-L-leucyl-*N*-methyl-L-leucyl-*N*-methyl-L-valyl] and having the chemical formula:

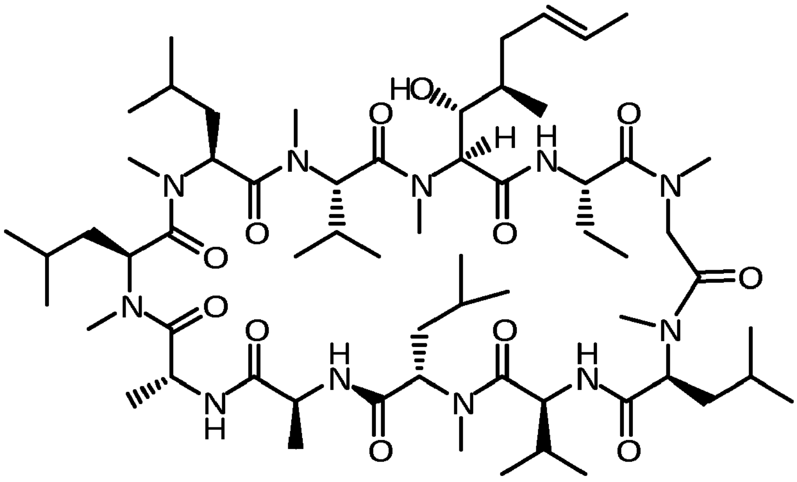

Cyclosporine A is a calcineurin phosphatase inhibitor, having potent immunosuppressive activity.

In a particular embodiment, the microemulsion of the present invention comprises from 0.1% w/w to 7% w/w by weight of cyclosporine A relative to the total weight of the microemulsion. In a preferred embodiment, the microemulsion of the present invention comprises from 1% w/w to 7% w/w of cyclosporine A with respect to the total weight of the microemulsion, preferably from 1% w/w to 5% w/w, more preferably from 1.5% w/w to 7% w/w by weight, still more preferably from 1.5% w/w to 5% w/w, even more preferably in a concentration from 2% w/w to 5% w/w.

Component b) in the pharmaceutical microemulsions of the present invention is a half $C_{1-4}$-alkyl ester of a poly (methyl vinyl ether-co-maleic anhydride) (PVM/MA) copolymer.

As used herein, the term "$C_{1-4}$-alkyl" refers to a linear or branched saturated monovalent hydrocarbon chain containing 1 to 4 carbon atoms, such as methyl, ethyl, propyl, isopropyl, butyl, isobutyl, sec-butyl, tert-butyl, etc.

The terms "half $C_{1-4}$-alkyl ester of a poly (methyl vinyl ether-co-maleic anhydride) (PVM/MA) copolymer" or "half $C_{1-4}$-alkyl ester of a PVM/MA copolymer" are used interchangeably here and refer to water-insoluble copolymers that are water-soluble when neutralized by bases in aqueous solution and having a structure of formula

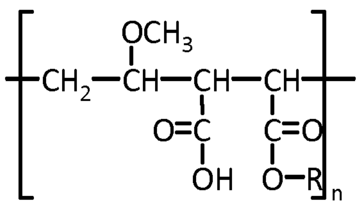

wherein R is a $C_{1-4}$-alkyl, i.e. PVM/MA copolymer ester in which only one of the two carboxyl groups is esterified. These half esters include the half ester form of PVM/MA with different alkyl chain lengths (such as monoethyl ester, wherein R is ethyl; monobutyl ester, wherein R is butyl; and isopropyl ester, wherein R is isopropyl). Said copolymers are commercialized by International Specialty Products (ISP) under trademark Gantrez® ES and include Gantrez® ES 225 (monoethyl ester), Gantrez® ES 425 (monobutyl ester) and Gantrez® ES335I (isopropyl ester) and are supplied as alcoholic solutions, for example, in ethanolic solutions [50% (w/v)].

In a particular embodiment, the half $C_{1-4}$-alkylalkyl ester of a PVM/MA copolymer is selected from the group consisting of ethyl ester of a PVM/MA copolymer, isopropyl ester of a PVM/MA copolymer and n-butyl ester of a PVM/MA copolymer; more preferably n-butyl ester of a PVM/MA copolymer.

In a preferred embodiment, the microemulsion of the invention comprises from 0.02 to 5% w/w of a half $C_{1-4}$-alkyl alkyl ester of a PVM/MA copolymer, preferably from 0.02to2% w/w, more preferably from 0.05to 1.8% w/w.

In the pharmaceutical compositions of the invention the half $C_{1-4}$-alkylalkyl ester of a PVM/MA copolymer is dissolved in a volatile alcohol, in a non-volatile organic solvent other than 2-(2-ethoxyethoxy)ethanol and surfactants having an HLB value from 10 to 18, or in a mixture of one or several volatile alcohol and one or several non-volatile organic solvent other than 2-(2-ethoxyethoxy)ethanol and surfactants having an HLB value from 10 to 18, i.e. in components c) and d) of the microemulsion of the invention.

The term "non-volatile organic solvent", as used herein, refers to an organic liquid that does not evaporate easily or evaporates very slowly at room temperature, i.e. that has vapor pressure lower than 1 kPa at 25 °C and boiling point higher than 110 °C at standard atmospheric pressure (101.325 kPa), but which does not include 2-(2-ethoxyethoxy)ethanol, and surfactants having an HLB value from 10 to 18. The non-volatile organic solvents, other than 2-(2-ethoxyethoxy)ethanol and surfactants having an HLB value from 10 to 18 useful in the present invention, must be capable of solubilizing 10 mg of the half$C_{1-4}$-alkylalkyl ester of a PVM/MA copolymer per mL of solvent. 2-(2-ethoxyethoxy)ethanol and some surfactants having an HLB value from 10 to 18 are also capable of solubilizing the copolymer, but the compositions of the present invention need an organic solvent different from these. Thus, 2-(2-ethoxyethoxy)ethanol and some surfactants having an HLB value from 10 to 18 are not considered within the scope of non-volatile organic solvent in the compositions of the present invention and, therefore, they are explicitly excluded from this category (component (c)). Exemplary non-volatile organic solvents other than 2-(2-ethoxyethoxy)ethanol and surfactants having an HLB value from 10 to 18, which are capable of solubilizing a half $C_{1-4}$-alkylalkyl ester of a PVM/MA copolymer that can be used in the present invention include, without limitation, propylene glycol and polyethylene glycol. Preferably, the non-volatile organic solvent other than 2-(2-ethoxyethoxy)ethanol and surfactants having an HLB value from 10 to 18, i.e. component c),is propylene glycol. The non-volatile organic solvent other than 2-(2-ethoxyethoxy)ethanol and surfactants having an HLB value from 10 to 18, also encompasses a mixture of one or more non-volatile organic solvents as herein defined, such as a mixture of one, two or three non-volatile organic solvents as herein defined, preferably only one non-volatile organic solvent other than 2-(2-ethoxyethoxy)ethanol and surfactants having an HLB value from 10 to 18.

In a particular embodiment the microemulsion of the invention comprises from 5% w/w to 15% w/w of a non-volatile organic solvent other than 2-(2-ethoxyethoxy)ethanol and surfactants having an HLB value from 10 to 18 capable of solubilizing the polymer, preferably from 10% w/w to 15% w/w (in particular when the microemulsion is water-free) or alternatively from 5% w/w to 10% w/w (in particular when the microemulsion contains more than 10% of water), more preferably from 12% w/w to 13% w/w (in particular when the microemulsion is water-free) or alternatively from 5.0% w/w to 8.5% w/w (in particular when the microemulsion is water-free).

In a preferred embodiment the non-volatile organic solvent other than 2-(2-ethoxyethoxy)ethanol and surfactants having an HLB value from 10 to 18 is propylene glycol. The term "propylene glycol", refers to propane-1,2-diol.

In a particular embodiment the microemulsion of the invention comprises from 5% w/w to 15% w/w of propylene glycol, preferably from 10% w/w to 15% w/w of propylene glycol(in particular when the microemulsion is water-free) or alternatively from 5% w/w to 10% w/w of propylene glycol(in particular when the microemulsion contains more than 10% of water), more preferably from 12% w/w to 13% w/w of propylene glycol (in particular when the microemulsion is water-free) or alternatively from 5.0% w/w to 8.5% w/w of propylene glycol (in particular when the microemulsion contains more than 10% of water).

Component d) in the pharmaceutical microemulsions of the present invention is a volatile alcohol.

A "volatile alcohol", as used herein, refers to a liquid alcohol that vaporizes/evaporates easily at room temperature; a volatile alcohol usually has a vapor pressure higher than 1 kPa at 25 °C and a boiling point lower than 110 °C. The volatile alcohols useful in the present invention are capable of solubilizing 10 mg of the half $C_{1-4}$-alkylalkyl ester of a PVM/MA copolymer per mL of volatile alcohol. The term "alcohol" refers to a linear or branched saturated monovalent hydrocarbon chain containing the indicated number of carbon atoms, typically from 1 to 4 carbon atoms, preferably from 1 to 3 carbon atoms, said hydrocarbon chain being linked to an OH group. Examples of volatile alcohols are methanol, ethanol, isopropanol, isobutanol, etc.

In a particular embodiment the microemulsion of the invention comprises from 0.05% w/w to 15% w/w of a volatile alcohol, preferably from 5% w/w to 15% w/w of a volatile alcohol (in particular when the microemulsion is water-free) or alternatively from 1% w/w to 10% w/w of a volatile alcohol (in particular when the microemulsion contains more than 10% of water), more preferably from 1% w/w to 6% w/w of a volatile alcohol (in particular when the microemulsion contains more than 10% of water) or alternatively from 7% w/w to 10% w/w of a volatile alcohol (in particular when the microemulsion is water-free), still more preferably from 2% w/w to 5.5% w/w of a volatile alcohol (in particular when the microemulsion contains more than 10% of water) or alternatively from 9% w/w to 10% w/w of a volatile alcohol (in particular when the microemulsion is water-free).

In a preferred embodiment the volatile alcohol is ethanol.

In a particular embodiment the microemulsion of the invention comprises from 0.05% w/w to 15% w/w of ethanol, preferably from 5% w/w to 15% w/w of ethanol (in particular when the microemulsion is water-free) or alternatively from 1% w/w to 10% w/w of ethanol (in particular when the microemulsion contains more than 10% of water), more preferably from 1% w/w to 6% w/w of ethanol (in particular when the microemulsion contains more than 10% of water) or alternatively from 7% w/w to 10% w/w of ethanol (in particular when the microemulsion is water-free), still more preferably from 2% w/w to 5.5% w/w of ethanol (in particular when the microemulsion contains more than 10% of water) or alternatively from 9% w/w to 10% w/w of ethanol (in particular when the microemulsion is water-free).

Component e) in the pharmaceutical microemulsions of the present invention is 2-(2-ethoxyethoxy)ethanol.

2-(2-Ethoxyethoxy)ethanol is commercialized under the name transcutol.

In a particular embodiment the microemulsion of the invention comprises from 15% w/w to 25% w/w of 2-(2-ethoxyethoxy)ethanol, preferably from 20% w/w to 25% w/w of 2-(2-ethoxyethoxy)ethanol, more preferably from 20% w/w to 23% w/w of 2-(2-ethoxyethoxy)ethanol.

Component f) in the pharmaceutical microemulsions of the present invention isa medium chain triglyceride.

The term "medium-chain triglyceride" or "MCT", as used herein, refers to triglycerides triesters of glycerol and 6-12 carbon fatty acid. The fatty acids found in medium-chain triglycerides are called medium-chain fatty acids. Like all triglycerides (fats and oils), medium-chain triglycerides are composed of a glycerol backbone and three fatty acids. In the case of medium-chain triglycerides, 2 or 3 of the fatty acid chains attached to glycerol are medium-chain in length. The three fatty acids of the MCT can be the same or different, preferably there are two different fatty acids. Examples of medium fatty acids are caproic or hexanoic acid (C6:0), caprylic or octanoic acid (C8:0), capric or decanoic acid (C10:0) and lauric or dodecanoic acid (C12:0).

The presence of a MCT in the microemulsion of the invention is particularly advantageous since it contributes to the stability of the formulations. As shown in the examples, the use of other oils(such as oleic acid, isopropyl myristate, ethyl oleate or triolein), particularly in the absence of MCT does not allow obtaining stable microemulsions both in terms of physical stability and chemical stability.

In a particular embodiment the microemulsion of the invention comprises from 2% w/w to 30% w/w of a MCT, preferably from 10% w/w to 30% w/w of a MCT (in particular when the microemulsion is water-free) or alternatively from 2% w/w to 10% w/w of a MCT (in particular when the microemulsion contains more than 10% of water), more preferably from 15% w/w to 25% w/w of a MCT (in particular when the microemulsion is water-free) or alternatively from 2% w/w to 8% w/w of a MCT (in particular when the microemulsion contains more than 10% of water), still more preferably from 20% w/w to 21% w/w of a MCT (in particular when the microemulsion is water-free) or alternatively from 4% w/w to 5% w/w of a MCT (in particular when the microemulsion contains more than 10% of water).

In a preferred embodiment, the medium chain triglyceride is caprylic/capric acid triglyceride.

In a particular embodiment the microemulsion of the invention comprises from 2% w/w to 30% w/w of caprylic/capric acid triglyceride, preferably from 10% w/w to 30% w/w of caprylic/capric acid triglyceride (in particular when the microemulsion is water-free) or alternatively from 2% w/w to 10% w/w of caprylic/capric acid triglyceride (in particular when the microemulsion contains more than 10% of water), more preferably from 15% w/w to 25% w/w of caprylic/capric acid triglyceride (in particular when the microemulsion is water-free) or alternatively from 2% w/w to 8% w/w of caprylic/capric acid triglyceride (in particular when the microemulsion contains more than 10% of water), still more preferably from 20% w/w to 21% w/w of caprylic/capric acid triglyceride (in particular when the microemulsion is water-free) or alternatively from 4% w/w to 5% w/w of caprylic/capric acid triglyceride (in particular when the microemulsion contains more than 10% of water).

Component g) in the pharmaceutical microemulsions of the present invention isa surfactant or surfactant mixture having an HLB value from 10 to 18.

The term "surfactant", as used herein, refers to a compound that lowers the surface tension or interfacial tension between two liquids or between a liquid and a solid. Surfactants may act as detergents, wetting agents, emulsifiers, foaming agents and dispersants.

The term "HLB" refers to the hydrophilic-lipophilic balance and is a measure of the degree to which a surfactant is hydrophilic or lipophilic. The HLB values of surfactants are widely reported in the literature [see for example Griffin, Journal of the Society of Cosmetic Chemists, 1949, 1, 311-326; Raymond C Rowe, Poul, J. Sheskey, Marian E Quinn (Eds). Handbook of pharmaceutical excipients. Pubhlished by the Pharmaceutical Press and the American Pharmacists Association. Sixth edition, 2009]. When two or more surfactants are present in the microemulsion of the invention, the total $HLB_t$ value of the mixture of said two or more nonionic surfactants is calculated as the weight average of the HLB values of the two or more nonionic surfactants (see following equation (1)).

$$HLB_t = (\sum W_i \cdot HLB_i) / (\sum W_i) \qquad \text{Equation (1)}$$

wherein $W_i$ and $HLB_i$ indicate the weight and the HLB value of the i-th nonionic surfactant, respectively

Surfactants having an HLB value from 10 to 18that can be used in the present invention are, among others, polysorbates and poloxamers.

Polysorbates refer to esters of sorbitan with fatty acids such as lauric acid, palmitic acid, stearic acid and oleic acid. The number following the polysorbate part is related to the type of fatty acid associated with the polyoxyethylene sorbitan part of the molecule. Monolaurate is indicated by 20, monopalmitate is indicated by 40, monostearate by 60 and monooleate by 80. Non-limiting examples of polysorbates having an HLB value from 10 to 18 that can be used in the present invention arepolysorbate-20, polysorbate-21, polysorbate-40, polysorbate-60, polysorbate-65, polysorbate-80 and polysorbate-81.

Poloxamers refer to polyoxyethylene-polyoxypropylene copolymers. Non-limiting examples of poloxamers having an HLB value from 10 to 18 that can be used in the present invention are poloxamer 184 and poloxamer 185.The particular poloxamers are followed by a number, the first two digits of which, when multiplied by 100, correspond to the approximate average molecular weight of the polyoxypropylene portion of the copolymer, and the third digit, when multiplied by 10, corresponds to the percentage by weight of the polyoxyethylene portion.

Mixtures of surfactants can also be used.

In a particular embodiment the microemulsion of the invention comprises from 10% w/w to 50% w/w of a surfactant or surfactant mixture having an HLB value from 10 to 18, preferably from 10% w/w to 30% w/w of a surfactant or surfactant mixture having an HLB value from 10 to 18 (in particular when the microemulsion is water-free) or alternatively from 20% w/w to 50% w/w of a surfactant or surfactant mixture having an HLB value from 10 to 18 (in particular when the microemulsion contains more than 10% of water), more preferably from 10% w/w to 20% w/w of a surfactant or surfactant mixture having an HLB value from 10 to 18 (in particular when the microemulsion is water-free) or alternatively from 35% w/w to 45% w/w of a surfactant or surfactant mixture having an HLB value from 10 to 18 (in particular when the microemulsion contains more than 10% of water).

In a preferred embodiment, the surfactant is polysorbate 80. The term "polysorbate 80", also known as Tween 80, refers to a nonionic surfactant and emulsifier often used in foods and cosmetics. The synthetic compound is a viscous, water-soluble yellow liquid derived from polyethoxylated sorbitan and oleic acid. Its full chemical name is polyoxyethylene (20) sorbitan monooleate or (x)-sorbitan mono-9-octadecenoate poly(oxy-1,2-ethanediyl). Its chemical formula is:

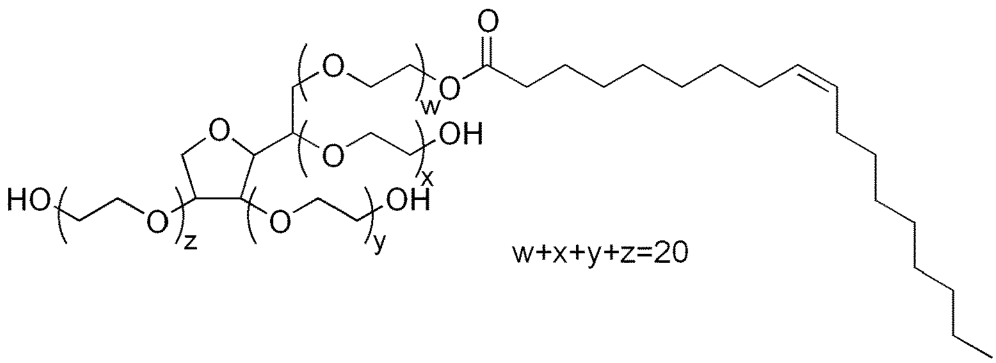

In a particular embodiment the microemulsion of the invention comprises from 10% w/w to 50% w/w of polysorbate 80, preferably from 10% w/w to 20% w/w polysorbate 80 (in particular when the microemulsion is water-free) or alternatively from 20% w/w to 30% w/w of polysorbate 80 (in particular when the microemulsion contains more than 10% of water), more preferably from 10% w/w to 15% w/w of a polysorbate 80 (in particular when the microemulsion is water-free) or alternatively from 22% w/w to 30% w/w of polysorbate 80 (in particular when the microemulsion contains more than 10% of water).

In another preferred embodiment the surfactant is a mixture of polysorbate 80 and polysorbate 20, in particular when the microemulsion contains more than 10% of water.

The term "polysorbate 20", also known as Tween 20, refers to a nonionic surfactant and emulsifier often used in foods and cosmetics. The synthetic compound is a viscous, water-soluble yellow liquid derived from polyethoxylated sorbitan and lauric acid. Its full chemical name is polyoxyethylene (20) sorbitan monolaurate. Its chemical formula is:

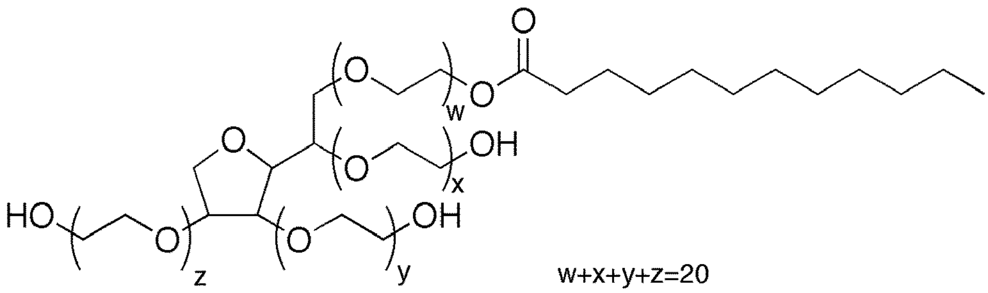

In a particular embodiment the microemulsion of the invention comprises from 20% w/w to 30% w/w of polysorbate 80 and from 10% w/w to 20% w/w of polysorbate 20 (in particular when the microemulsion contains more than

10% of water), more preferably from 25% w/w to 30% w/w of polysorbate 80, and from 10% w/w to 15% w/w of polysorbate 20 (in particular when the microemulsion contains more than 10% of water).

Component h), i.e. water, is optionally present in the pharmaceutical microemulsions of the present invention.

In one embodiment water is present in the microemulsions of the invention. In a particular embodiment the microemulsion of the invention comprises more than 10% w/w of water, preferably from 15% w/w to 25% w/w of water, more preferably from 17% w/w to 21% w/w of water. In another embodiment, the microemulsion of the invention is water-free.

The term "water-free" when characterizing the microemulsion of the invention refers microemulsions substantially free from water. However, the water-free microemulsion of the invention may contain non-significant quantities of water coming from the rest of the components of the formulation. Particularly, the water-free microemulsion of the invention does not comprise more than 2% w/w of water. The water-free microemulsion of the invention may comprise between 0% w/w and 2% w/w of water. In particular, unless explicit reference is made to the presence of water, the microemulsions of the invention are substantially free from water, i.e. do not comprise more than 2% w/w of water. The water-free microemulsions of the invention have particularly advantageous organoleptic properties and extensibility on the skin.

Component i) is optionally present in the pharmaceutical microemulsions of the present invention and is triacetin.

The term "triacetin", as used herein, is the triglyceride 1,2,3-triacetoxypropane and is also known as glycerin triacetate or 1,3-diacetyloxypropan-2-yl-acetate and its chemical formula is the following:

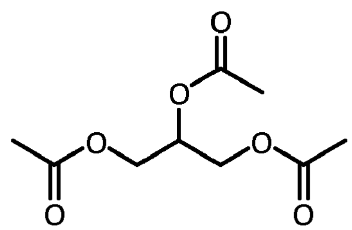

In one embodiment triacetin is present in the microemulsions of the invention. In a particular embodiment the microemulsion of the invention comprises from 10% w/w to 20% w/w of triacetin(in particular when the microemulsion is water-free), more preferably from 15% w/w to 20% w/w of triacetin(in particular when the microemulsion is water-free).Preferably, water is not present in the microemulsions of the invention comprising triacetin. Preferably, triacetin is not present in the microemulsions of the invention comprising water.

In a preferred embodiment, the microemulsion of the present invention comprises:

(a) from 1% w/w to 7% w/w of cyclosporine A,
(b) from 0.02% w/w to 5% w/w of a half $C_{1-4}$-alkyl ester of a poly(methyl vinyl ether-co-maleic anhydride) (PVM/MA) copolymer,
(c) from 5% w/w to 15% w/w of a non-volatile organic solvent capable of solubilizing component (b)other than 2-(2-ethoxyethoxy)ethanol and surfactants having an HLB value from 10 to 18,
(d) from 0.05% w/w to 15% w/w of a volatile alcohol,
(e) from 20% w/w to 25% w/w of 2-(2-ethoxyethoxy)ethanol,
(f) from 2% w/w to 30% w/w of a medium chain triglyceride,
(g) from 10% w/w to 50% w/w of a surfactant or surfactant mixture having an HLB value from 10 to 18,
(h) optionally from 15% w/w to 25% w/w of water, and
(i) optionally from 10% w/w to 20% w/w of triacetin,

wherein w/w is the weight of each component relative to the total weight of the microemulsion.

In another preferred embodiment, the microemulsion of the present invention comprises:

(a) from 1% w/w to 7% w/w of cyclosporine A,
(b) from 0.02% w/w to 5% w/w of a half $C_{1-4}$-alkyl ester of a poly(methyl vinyl ether-co-maleic anhydride) (PVM/MA) copolymer,
(c) from 5% w/w to 15% w/w of a non-volatile organic solvent capable of solubilizing component (b)other than 2-(2-ethoxyethoxy)ethanol and surfactants having an HLB value from 10 to 18,
(d) from 0.05% w/w to 15% w/w of a volatile alcohol,
(e) from 20% w/w to 25% w/w of 2-(2-ethoxyethoxy)ethanol,
(f) from 2% w/w to 30% w/w of a medium chain triglyceride,

(g) from 10% w/w to 50% w/w of a surfactant or surfactant mixture having an HLB value from 10 to 18, and
(h) from 15% w/w to 25% w/w of water,

wherein w/w is the weight of each component relative to the total weight of the microemulsion.

In another preferred embodiment, the microemulsion of the present invention comprises:

(a) from 1% w/w to 7% w/w of cyclosporine A,
(b) from 0.02% w/w to 5% w/w of a half $C_{1-4}$-alkyl ester of a poly(methyl vinyl ether-co-maleic anhydride) (PVM/MA) copolymer,
(c) from 5% w/w to 15% w/w of a non-volatile organic solvent capable of solubilizing component (b)other than 2-(2-ethoxyethoxy)ethanol and a surfactants having an HLB value from 10 to 18,
(d) from 0.05% w/w to 15% w/w of a volatile alcohol,
(e) from 20% w/w to 25% w/w of 2-(2-ethoxyethoxy)ethanol,
(f) from 2% w/w to 30% w/w of a medium chain triglyceride,
(g) from 10% w/w to 50% w/w of a surfactant or surfactant mixture having an HLB value from 10 to 18, and
(i) optionally from 10% w/w to 20% w/w of triacetin,

wherein w/w is the weight of each component relative to the total weight of the microemulsion.

In another preferred embodiment, the microemulsion of the present invention comprises:

(a) from 1% w/w to 7% w/w of cyclosporine A,
(b) from 0.02% w/w to 5% w/w of a half $C_{1-4}$-alkylester of a poly(methyl vinyl ether-co-maleic anhydride) (PVM/MA) copolymer,
(c) from 5% w/w to 15% w/w of a non-volatile organic solvent capable of solubilizing component (b)other than 2-(2-ethoxyethoxy)ethanol and surfactants having and HLB value from 10 to 18,
(d) from 5% w/w to 15% w/w of a volatile alcohol,
(e) from 20% w/w to 25% w/w of 2-(2-ethoxyethoxy)ethanol,
(f) from 10% w/w to 30% w/w of a medium chain triglyceride,
(g) from 10% w/w to 20% w/w of a surfactant or surfactant mixture having an HLB value from 10 to 18, and
(i) from 10% w/w to 20% w/w of triacetin,

wherein w/w is the weight of each component relative to the total weight of the microemulsion.

In another preferred embodiment, the microemulsion of the present invention comprises:

(a) from 1% w/w to 7% w/w of cyclosporine A,
(b) from 0.02% w/w to 5% w/w of a half $C_{1-4}$-alkyl ester of a poly(methyl vinyl ether-co-maleic anhydride) (PVM/MA) copolymer,
(c) from 10% w/w to 15% w/w of a non-volatile organic solvent capable of solubilizing component (b)other than 2-(2-ethoxyethoxy)ethanol and surfactant having an HLB value from 10 to 18,
(d) from 5% w/w to 15% w/w of a volatile alcohol,
(e) from 20% w/w to 25% w/w of 2-(2-ethoxyethoxy)ethanol,
(f) from 15% w/w to 25% w/w of a medium chain triglyceride,
(g) from 10% w/w to 15% w/w of a surfactant or surfactant mixture having an HLB value from 10 to 18, and
(i) from 15% w/w to 20% w/w of triacetin,

wherein w/w is the weight of each component relative to the total weight of the microemulsion.

In another preferred embodiment, the microemulsion of the present invention comprises:

(a) from 1% w/w to 7% w/w of cyclosporine A,
(b) from 0.02% w/w to 1% w/w of the half n-butyl ester of a PVM/MA copolymer,
(c) from 5% w/w to 15% w/w of propylene glycol,
(d) from 5% w/w to 15% w/w of ethanol,
(e) from 20% w/w to 25% w/w of 2-(2-ethoxyethoxy)ethanol,
(f) from 10% w/w to 30% w/w of caprylic/capric acid triglyceride,
(g) from 10% w/w to 20% w/w polysorbate 80, and
(i) from 10% w/w to 20% w/w of triacetin,

wherein w/w is the weight of each component relative to the total weight of the microemulsion.

In another preferred embodiment, the microemulsion of the present invention comprises:

(a) from 1% w/w to 7% w/w of cyclosporine A,
(b) from 0.02% w/w to 1% w/w of the half n-butyl ester of a PVM/MA copolymer,
(c) from 10% w/w to 15% w/w of propylene glycol,
(d) from 5% w/w to 15% w/w of ethanol,
(e) from 20% w/w to 25% w/w of 2-(2-ethoxyethoxy)ethanol,
(f) from 15% w/w to 25% w/w of caprylic/capric acid triglyceride,
(g) from 10% w/w to 15% w/w polysorbate 80, and
(i) from 15% w/w to 20% w/w of triacetin,

wherein w/w is the weight of each component relative to the total weight of the microemulsion.
In another preferred embodiment, the microemulsion of the present invention comprises:

(a) from 1% w/w to 7% w/w of cyclosporine A,
(b) from 0.02% w/w to 0.1% w/w of the half n-butyl ester of a PVM/MA copolymer,
(c) from 12% w/w to 13% w/w of propylene glycol,
(d) from 8% w/w to 10% w/w of ethanol,
(e) from 20% w/w to 23% w/w of 2-(2-ethoxyethoxy)ethanol,
(f) from 20% w/w to 22% w/w of caprylic/capric acid triglyceride,
(g) from 10% w/w to 15% w/w polysorbate 80, and
(i) from 15% w/w to 20% w/w of triacetin,

wherein w/w is the weight of each component relative to the total weight of the microemulsion.
In another preferred embodiment, the microemulsion of the present invention comprises:

(a) from 1% w/w to 5% w/w of cyclosporine A,
(b) from 0.02% w/w to 5% w/w of a half $C_{1-4}$-alkylester of a poly(methyl vinyl ether-co-maleic anhydride) (PVM/MA) copolymer,
(c) from 5% w/w to 15% w/w of a non-volatile organic solvent capable of solubilizing component (b)other than 2-(2-ethoxyethoxy)ethanol and surfactants having an HLB value from 10 to 18,
(d) from 1% w/w to 10% w/w of a volatile alcohol,
(e) from 20% w/w to 25% w/w of 2-(2-ethoxyethoxy)ethanol,
(f) from 2% w/w to 10% w/w of a medium chain triglyceride,
(g) from 30% w/w to 50% w/w of a surfactant or surfactant mixture having an HLB value from 10 to 18, and
(h) from 15% w/w to 25% w/w of water,

wherein w/w is the weight of each component relative to the total weight of the microemulsion.
In another preferred embodiment, the microemulsion of the present invention comprises:

(a) from 1% w/w to 5% w/w of cyclosporine A,
(b) from 0.02% w/w to 5% w/w of a half $C_{1-4}$-alkyl ester of a poly(methyl vinyl ether-co-maleic anhydride) (PVM/MA) copolymer,
(c) from 5% w/w to 10% w/w of a non-volatile organic solvent capable of solubilizing component (b)other than 2-(2-ethoxyethoxy)ethanol and surfactants having an HLB value from 10 to 18,
(d) from 1% w/w to 6% w/w of a volatile alcohol,
(e) from 20% w/w to 25% w/w of 2-(2-ethoxyethoxy)ethanol,
(f) from 2% w/w to 8% w/w of a medium chain triglyceride,
(g) from 35% w/w to 45% w/w of a surfactant or surfactant mixture having an HLB value from 10 to 18, and
(h) from 17% w/w to 21% w/w of water,

wherein w/w is the weight of each component relative to the total weight of the microemulsion.
In another preferred embodiment, the microemulsion of the present invention comprises:

(a) from 1% w/w to 5% w/w of cyclosporine A,
(b) from 0.02% w/w to 5% w/w of the half n-butyl ester of a PVM/MA copolymer,
(c) from 5% w/w to 15% w/w of propylene glycol,
(d) from 1% w/w to 10% w/w of ethanol,

(e) from 20% w/w to 25% w/w of 2-(2-ethoxyethoxy)ethanol,
(f) from 2% w/w to 10% w/w of a caprylic/capric acid triglyceride,
(g) from 20% w/w to 30% w/w of polysorbate 80 and from 10% w/w to 20% w/w of polysorbate 20, and
(h) from 15% w/w to 25% w/w of water,

wherein w/w is the weight of each component relative to the total weight of the microemulsion.

In another preferred embodiment, the microemulsion of the present invention comprises:

(a) from 1% w/w to 5% w/w of cyclosporine A,
(b) from 0.02% w/w to 5% w/w of the half n-butyl ester of a PVM/MA copolymer,
(c) from 5% w/w to 10% w/w of propylene glycol,
(d) from 1% w/w to 10% w/w of ethanol,
(e) from 20% w/w to 25% w/w of 2-(2-ethoxyethoxy)ethanol,
(f) from 2% w/w to 8% w/w of a caprylic/capric acid triglyceride,
(g) from 20% w/w to 30% w/w of polysorbate 80 and from 10% w/w to 20% w/w of polysorbate 20, and
(h) from 15% w/w to 25% w/w of water,

wherein w/w is the weight of each component relative to the total weight of the microemulsion.

In another preferred embodiment, the microemulsion of the present invention comprises:

(a) from 1% w/w to 5% w/w of cyclosporine A,
(b) from 0.02% w/w to 2% w/w of the half n-butyl ester of a PVM/MA copolymer,
(c) from 7.5% w/w to 8.5% w/w of propylene glycol,
(d) from 1.5% w/w to 6% w/w of ethanol,
(e) from 20% w/w to 23% w/w of 2-(2-ethoxyethoxy)ethanol,
(f) from 4% w/w to 5% w/w of a caprylic/capric acid triglyceride,
(g) from 22% w/w to 30% w/w of polysorbate 80 and from 10% w/w to 15% w/w of polysorbate 20, and
(h) from 17% w/w to 21% w/w of water,

wherein w/w is the weight of each component relative to the total weight of the microemulsion.

In a particular embodiment, the microemulsion consists of the ingredients explicitly mentioned with respect to any of the embodiments described herein, i.e. they do not comprise ingredients other than those explicitly mentioned.

The microemulsions of the invention do not require the use of preservatives. Therefore, in another embodiment, the microemulsion does not contain additional preservatives. By "additional preservatives", as used herein, is understood as substances added to pharmaceutical products to prevent decomposition by microbial growth or by undesirable chemical changes. Additional preservatives include antimicrobial additives and antioxidants.

Although it is not required to add additional preservatives, the microemulsions of the invention may contain preservatives. Exemplary preservatives that can be used in the microemulsions of the invention include, without limitation, potassium sorbate, sodium benzoate, phenoxyethanol, sorbic acid, thimerosal, benzalkonium chloride, parabens, etc.

In another embodiment, the microemulsion does not contain oleic acid.

In another embodiment, the microemulsion does not contain additional preservatives and oleic acid.

The compositions of the present invention are stable and allow formulating high concentrations of cyclosporine A, without the use of a polyalkyl ester of polycarboxylic acid, such as adipic, pimelic, azelaic, sebacic and phthalic dialkyl esters.

The term "polyalkyl ester of polycarboxylic acid", as used herein, refers to the polyalkyl ester of polycarboxylic acids disclosed in US 5,891,846 (which is incorporated by reference with respect to their definition), and are defined as those which are liquid at ordinary temperature. The esters having a total carbon atom number of 10 to 25 are preferable and the esters having polycarboxylic acids having at least two carboxyl groups and linear or branched alcohols, in which the carboxyl group may be an aliphatic group, aromatic aliphatic group, or aromatic group having the carboxyl groups bonded aliphatically or aromatically are preferable. Partial ester compounds may also be used. These esters may be used alone or in a mixture of two kinds or more. Specific examples include adipic dialkyl esters of a total of 12 to 22 carbon atoms, pimelic dialkyl esters having a total of 13 to 23 carbon atoms, sebacic dialkyl esters having a total of 14 to 22 carbon atoms, phthalic dialkyl esters having a total of 14 to 24 carbon atoms (these alkyl groups may be straight or branched and the alkyl portion of the dialkyl may be the same or different). Preferable examples are dibutyl phthalate, diethyl phthalate, diisobutyl phthalate, dibutyl sebacate, diethyl sebacate, diisopropyl azelate, diisopropyl adipate, dibutyl adipate, and diisobutyl adipate.

Thus, in a particular embodiment, the microemulsion of the present invention does not contain a polyalkyl ester of polycarboxylic acid. In another particular embodiment, the microemulsion does not contain additional preservatives

and a polyalkyl ester of polycarboxylic acid.In another particular embodiment, the microemulsion does not contain oleic acid and a polyalkyl ester of polycarboxylic acid. In a further particular embodiment, the microemulsion does not contain oleic acid, additional preservatives and a polyalkyl ester of polycarboxylic acid.

The composition of the invention may contain excipients. The term "excipient", as used herein, refers to an inactive substance that can be liquid, solid or semisolid, used as a medium or carrier for the active ingredients of a composition. Illustrative, non-limitative examples of excipients are butyl hydroxytoluene (BHT), liquid paraffin or melted lipids such as wax, cotton oil, hydrogenated vegetable oil, canola oil, coconut oil, etc. Thickening agents, i.e. substances that increase the viscosity of the compositions, may also be added to the compositions of the invention. Said excipients are particularly useful in the production of microemulsions and they may be found in the disperse phase of said microemulsions.

The person skilled in the art knows that the microemulsions of the invention may be administered in the form of pharmaceutical compositions comprising cyclosporine A as a sole active ingredient or in combinations with other active ingredients.

The pharmaceutical compositions of the invention can be administered by different topical routes such as, without limitation, cutaneous, buccal, nasal or rectal route. In a preferred embodiment they are applied on the skin, i.e. cutaneous route, preferably by spraying. More preferably they are applied by massage.

Process for producing the pharmaceutical compositions

In the second aspect, the invention relates to a process for producing a topical pharmaceutical microemulsion as defined in the first aspect comprising:

(i) preparing an homogeneous mixture comprising cyclosporine A and the medium chain triglyceride,
(ii) preparing an homogeneous solution comprising the half $C_{1-4}$-alkyl ester of a poly(methyl vinyl ether-co-maleic anhydride) (PVM/MA) copolymer, the volatile alcohol and the non-volatile organic solvent other than 2-(2-ethoxyethoxy)ethanol and surfactants having an HLB value from 10 to 18;
(iii) adding the solution obtained in step (ii) to the mixture obtained in step (i) under stirring,
(iv) adding 2-(2-ethoxyethoxy)ethanol, the surfactant or surfactant mixture having an HLB value from 10 to 18, and optionally triacetin to the mixture obtained in step (iii) and stirring until a homogeneous solution is obtained, and
(v) optionally adding water to the mixture obtained in step (iv) and stirring until a homogeneous solution is obtained.

Step (i) comprises mixing cyclosporine A and the medium chain triglyceride and stir until complete dissolution of cyclosporine A or until an homogeneous suspension is obtained, preferably at room temperature (20-25°C). In particular the mixing is carried out for at least one hour.

Step (ii) comprises mixing the half $C_{1-4}$-alkyl ester of a poly(methyl vinyl ether-co-maleic anhydride) (PVM/MA) copolymer, the volatile alcohol and the non-volatile organic solvent other than 2-(2-ethoxyethoxy)ethanol and surfactants having an HLB value from 10 to 18 and stir until the copolymer is completely dissolved, preferably at room temperature (20-25°C).

Step (iii) comprises adding the copolymer solution obtained in step (ii) to the cyclosporine A mixture obtained in step (i) under stirring, preferably at room temperature (20-25°C).

Step (iv) comprises adding 2-(2-ethoxyethoxy)ethanol, the surfactant or surfactant mixture having an HLB value from 10 to 18, and optionally triacetin to the mixture obtained in step (iii) and stirring until a homogeneous solution is obtained, preferably at room temperature (20-25°C). If other excipients are present in the microemulsion of the invention, they are added at this step. At the end of step iv), a clear solution is obtained, which indicates that the microemulsion has been formed.

Finally, for those microemulsions of the invention comprising water, step (v) is performed. This step comprises adding water to the mixture obtained in step (iv) and stirring until a homogeneous solution is obtained, preferably at room temperature (20-25°C).

All the specific embodiments disclosed in the context of the compositions of the invention are applicable to the process of the invention.

Medical uses of the pharmaceutical compositions

The pharmaceutical compositions of the invention can be applied for the treatment of all diseases that can be topically treated with cyclosporine A, said diseases being disclosed in the background section of the present document.

Thus, in the third aspect, the invention relates to a topical pharmaceutical microemulsion of the invention for use as a medicament, in particular a medicament for human or veterinary use.

In a fourth aspect, the invention relates to a topical pharmaceutical microemulsion as defined in the first aspect

for use in the prevention and/or treatment of a disease selected from the group consisting of psoriasis, atopic dermatitis, allergic dermatitis, pyoderma gangrenosum, refractory chronic idiopathic urticaria, dyshidrotic eczema, Behçet disease, pityriasis rubra pilaris, dermatomyositis, pemphigus vulgaris, benign familiar pemphigus, pemphigus foliaceus and erythematosus, epidermolysis bullosa acquisita, photodermatoses, lichen planus, prurigo nodularis, alopecia areata, eosinophilic pustular folliculitis, granulomatous folliculitis and furunculosis, mular folliculitis, hidradenitis suppurativa, scleroderma, vitiligo, eosinophilic granuloma complex, perianal fistulas, sebaceous adenitis, juvenile cellulitis, vesicular cutaneous lupus erythematosus, erythema multiforme, discoid lupus erythematosus, sterile nodular panniculitis, metatarsal fistulae, nasal arteritis, ulcerative dermatosis of nasal philtrum, facial dermatitis, sterile granuloma or pyogranuloma syndrome, pseudopelade, cutaneous reactive histiocytosis, feline plasma cell pododermatitis, vasculitis and ischemic dermatopathy; preferably psoriasis, atopic dermatitis or allergic dermatitis.

In another aspect, the invention relates to the use of a topical pharmaceutical microemulsion of the invention for the manufacture of a medicament for the prevention and/or treatment of a disease selected from the group consisting of psoriasis, atopic dermatitis, allergic dermatitis, pyoderma gangrenosum, refractory chronic idiopathic urticaria, dyshidrotic eczema, Behçet disease, pityriasis rubra pilaris, dermatomyositis, pemphigus vulgaris, benign familiar pemphigus, pemphigus foliaceus and erythematosus, epidermolysis bullosa acquisita, photodermatoses, lichen planus, prurigo nodularis, alopecia areata, eosinophilic pustular folliculitis, granulomatous folliculitis and furunculosis, mular folliculitis, hidradenitis suppurativa, scleroderma, vitiligo, eosinophilic granuloma complex, perianal fistulas, sebaceous adenitis, juvenile cellulitis, vesicular cutaneous lupus erythematosus, erythema multiforme, discoid lupus erythematosus, sterile nodular panniculitis, metatarsal fistulae, nasal arteritis, ulcerative dermatosis of nasal philtrum, facial dermatitis, sterile granuloma or pyogranuloma syndrome, pseudopelade, cutaneous reactive histiocytosis, feline plasma cell pododermatitis, vasculitis and ischemic dermatopathy; preferably psoriasis, atopic dermatitis or allergic dermatitis.

In another aspect, the invention relates to a method of prevention and/or treatment of a subject suffering from a disease selected from the group consisting of psoriasis, atopic dermatitis, allergic dermatitis, pyoderma gangrenosum, refractory chronic idiopathic urticaria, dyshidrotic eczema, Behçet disease, pityriasis rubra pilaris, dermatomyositis, pemphigus vulgaris, benign familiar pemphigus, pemphigus foliaceus and erythematosus, epidermolysis bullosa acquisita, photodermatoses, lichen planus, prurigo nodularis, alopecia areata, eosinophilic pustular folliculitis, granulomatous folliculitis and furunculosis, mular folliculitis, hidradenitis suppurativa, scleroderma, vitiligo, eosinophilic granuloma complex, perianal fistulas, sebaceous adenitis, juvenile cellulitis, vesicular cutaneous lupus erythematosus, erythema multiforme, discoid lupus erythematosus, sterile nodular panniculitis, metatarsal fistulae, nasal arteritis, ulcerative dermatosis of nasal philtrum, facial dermatitis, sterile granuloma or pyogranuloma syndrome, pseudopelade, cutaneous reactive histiocytosis, feline plasma cell pododermatitis, vasculitis and ischemic dermatopathy, preferably psoriasis, atopic dermatitis or allergic dermatitis, comprising the administration to said subject of a topical pharmaceutical microemulsion of the invention.

In one particular embodiment of the aspects defined above, the disease is psoriasis.

In another particular embodiment of the aspects defined above, the disease is atopic dermatitis.

In another particular embodiment of the aspects defined above, the disease is allergic dermatitis.

In another particular embodiment of the aspects defined above, the disease is psoriasis or atopic dermatitis.

The term "prevention", as used herein, refers to the administration of the microemulsion of the invention in an initial or early stage of a disease, or to also prevent its onset.

The term "treatment" is used to designate the administration of the microemulsion of the invention to control disorder progression before or after the clinical signs had appeared. By control of the disorder progression it is meant to designate beneficial or desired clinical results including, but not limited to, reduction of symptoms, reduction of the length of the disorder, stabilization pathological state (specifically avoidance of further deterioration), delay in the disorder's progression, improvement of the pathological state and remission (both partial and total). In a particular embodiment of the invention the microemulsion of the invention is used to control the disorder progression once at least one of the disorder's clinical signs has appeared.

The term "medicament", as used herein, refers to a pharmaceutical microemulsion of the invention comprising cyclosporine A. The medicament may be administered by any suitable topical route. It is prepared by conventional means with pharmaceutically acceptable excipients. Formulations for application on the skin are preferred.

The term "subject", as used herein, refers to any animal or human that is suffering from one of the diseases disclosed above. Preferably, the subject is a mammal. The term "mammal", as used herein, refers to any mammalian species, including but not being limited to domestic and farm animals (cows, horses, pigs, sheep, goats, dogs, cats or rodents), primates, and humans. Preferably, the mammal is selected from a human being, a dog, a cat and a horse. In the context of the present invention, the mammal is suffering from a disease selected from the group consisting of psoriasis, atopic dermatitis, allergic dermatitis, pyoderma gangrenosum, refractory chronic idiopathic urticaria, dyshidrotic eczema, Behçet disease, pityriasis rubra pilaris, dermatomyositis, pemphigus vulgaris, benign familiar pemphigus, pemphigus foliaceus and erythematosus, epidermolysis bullosa acquisita, photodermatoses, lichen planus, prurigo nodularis, alopecia areata, eosinophilic pustular folliculitis, granulomatous folliculitis and furunculosis, mular folliculitis, hidradenitis

suppurativa, scleroderma, vitiligo, eosinophilic granuloma complex, perianal fistulas, sebaceous adenitis, juvenile cellulitis, vesicular cutaneous lupus erythematosus, erythema multiforme, discoid lupus erythematosus, sterile nodular panniculitis, metatarsal fistulae, nasal arteritis, ulcerative dermatosis of nasal philtrum, facial dermatitis, sterile granuloma or pyogranuloma syndrome, pseudopelade, cutaneous reactive histiocytosis, feline plasma cell pododermatitis, vasculitis and ischemic dermatopathy, preferably psoriasis, or in risk of suffering from one of said diseases.

All the embodiments disclosed in the context of the compositions of the invention are applicable to the medical uses of the pharmaceutical compositions of the invention.

The invention is described below by means of several examples which do not limit, but rather illustrate the invention.

## Examples

### 1. Materials

Cyclosporine A was purchased from Concorde Biothech Ltd. Caprylic/capric acid triglyceride (MCT), propylene glycol, polysorbate 80 (Tween® 80), polysorbate 20 (Tween®20) and isopropyl myristate (IPM) were purchased from Guinama. Gantrez® ES (poly(methyl vinyl ether-maleic acid monobutyl ester) (GES 425) and ethyl oleate were purchased from Sigma-Aldrich. 2-(2-ethoxyethoxy)ethanol P®was purchased from Fagron. Triacetin, ethanol absolute and oleic acid were purchased from Panreac. Acetonitrile HPLC grade was purchased from Merck.

### 2. Equipment

- Biological and cytostatic safety cabinet. Telstar, Cytostar, 29045.
- Analytical balance. Mettler Toledo, XA 204 Delta Range.
- Analytical balance. OHAUS, PA114C.
- Ultrasonic bath. Bandelin, Sonorex Digitec DT100H.
- Heating Stove. INDELAB, IDL-CD-120.
- Climatic chambers MEMMERT, HPP 108.
- Water purification system. Millipore, Direct Q 3UV.
- Autoclave. Raypa, AH-21N2.

### 3. Cyclosporine A formulations for topical application

The following formulations were prepared:

| | *Comparative examples* | | | | | *Examples* | | |
|---|---|---|---|---|---|---|---|---|
| Component % (w/w) | Ex. 1 (P49) | Ex. 2 (P63) | Ex. 3 (P64) | Ex. 4 (92A -E) | Ex. 5 (131A -D) | Ex. 6(P43 ) | Ex. 7(P51) | Ex. 8(P48) |
| EtOH | 1.5 | 1.9 | 1.6 | 1.8 | - | 3.6 | 2.0 | 9.3 |
| GES425* | 0.2 | 0.5 | 0.5 | 0.2 | 0.1 | 3.6 | 0.1 | 0.1 |
| 2-(2-ethoxyethoxy)ethanol | 18.8 | 18.1 | 18.0 | 22.7 | - | 20.3 | 22.5 | 22.8 |
| Polysorbate 20 | - | - | - | 28.2 | 11.8 | 13.2 | 13.7 | - |
| Propylene glycol | 5.4 | 5.3 | 6.9 | - | 10.8 | 8.0 | 7.8 | 12.4 |
| Polysorbate 80 | 22.7 | 22.8 | 22.1 | - | 12.0 | 25.4 | 27.4 | 13.0 |
| Oleic acid | - | - | - | 5.0 | 9.4 | - | - | - |
| Ethyl oleate | 4.0 | 4.1 | - | - | - | - | - | - |
| MCT | - | - | - | - | - | 4.3 | 4.9 | 20.8 |
| IPM | - | - | 4.1 | - | - | - | - | - |
| Dimethyl isosorbide | - | - | - | - | 7.6 | - | - | - |

(continued)

| | Comparative examples | | | | | Examples | | |
|---|---|---|---|---|---|---|---|---|
| Component % (w/w) | Ex. 1 (P49) | Ex. 2 (P63) | Ex. 3 (P64) | Ex. 4 (92A -E) | Ex. 5 (131A -D) | Ex. 6(P43 ) | Ex. 7(P51) | Ex. 8(P48) |
| Triacetin | 6.4 | 6.4 | 6.3 | - | - | - | - | 16.6 |
| Water | 39.5 | 39.4 | 39.5 | 40.6 | 47.2 | 19.6 | 19.6 | - |
| Cyclosporin A | 1.5 | 1.5 | 1.0 | 1.5 | 1.1 | 2.0 | 2.0 | 5.0 |
| * GES425 refers to the commercial Gantrez® ES (poly(methyl vinyl ether-maleic acid monobutyl ester) which is a solution of said polymer 50% w/w in ethanol. | | | | | | | | |

5 g of the formulations of Examples 1-8 were manufactured according to the following process:

- Preparation of the drug solution: the necessary amount of Cyclosporine A was weighed and then the corresponding amount of oil (i.e. oleic acid, ethyl oleate, MCT or IPM) was added. This solution was kept under magnetic stirring at room temperature for at least one hour.
- Polymer solution: The appropriate amount of GES425 was mixed with the necessary amount of ethanol and propylene glycol under continuous stirring until the polymer was completely dissolved.
- Excipient addition: To the previously described drug solution, the polymer solution was added under continuous stirring. Then, the rest of the components were added as required per the formulations composition but in this order: 2-(2-ethoxyethoxy)ethanol, tween 20, 80 and triacetine and/or dimethyl isosorbide. The mixture was kept under vigorous magnetic stirring at room temperature until a clear and homogeneous solution was obtained
- Aqueous mixture: When applicable, the corresponding amount of water was added to the previous mixture. The final formulation was kept under magnetic stirring at room temperature until a homogeneous mixture was achieved.

In addition, 100 g of the formulation of Example 6 and 200 g of the formulations of Examples 7-8 were also manufactured following the same procedure as described above.

These formulations showed appropriate properties to be administered by spraying.

**4. Stability studies**

*Physical stability.*

The cyclosporine A-loaded topical polymeric formulations described previously in Examples 6-8(1-3 mL)were kept for one week at 40 °C and 75% RH. Then they were subjected to stress conditions, i.e. three freezing cycles of 15 hours at -20 °C followed by 2 h at 80 °C. All the formulations were stable by macroscopical examination (i.e. the formulations remained transparent).

The cyclosporine A-loaded topical polymeric formulations described previously (5 g) were kept at 25 °C and 60% RH, at 40 °C and 75% RH. A loss of stability (presence of a white cloudy precipitate) was observed for formulation of example 1 after 5 days of storage at 25 °C and 60% RH and at 40 °C and 75% RH. A loss of stability (presence of a white cloudy precipitate) was also observed for formulations of examples 2 and 3 after 13 and 19 days, respectively, of storage at 25 °C and 60% RH. A loss of stability was also observe for formulation of example 5, which became slightly orange and lost viscosity after 6 weeks at 40 °C and 75% RH. Formulations of examples 4 and 6-8 remained stable after 8 weeks and also after 6 months (long-term stability) by macroscopical examination (i.e. the formulations remained transparent).

*Chemical stability*

The cyclosporine A-loaded topical polymeric formulations described previously (5 g) were kept at 40 °C and 75% RH for six weeks. The formulations were analyzed by HPLC chromatography before and after the six week storage.

HPLC chromatographic conditions:

- analytical column: Agilent Zorbax SB-C18 (250 x 4.6 mm) 5 μm,
- column temperature: 70 °C,

- mobile phase: ACN:$H_2O$ (75:25),
- flow: 2 mL/min,
- injection volume: 5 μL followed by a needle wash with mobile phase,
- detection wavelength: 210 nm using a high performance flow cell with a 60 mm flow path.

The relative error was calculated for each formulation as the concentration of cyclosporine A before storage with respect to the concentration after the period of storage and expressed as %. Formulations having a relative error of less than 5% were considered as chemically stable. Formulations having a relative error of 5% or more were considered as chemically unstable.

The formulation of examples 4 and 5were unstable after the 6-week storage whereas formulations of examples 6-8 remained stable after the6-week storage and also after a 6-monnth storage at 25°C and 60% RH and 40 °C and 75% RH.

**5. Percutaneous absorption**

Percutaneous absorption studies in Franz diffusion cells were performed to evaluate the degree of penetration of cyclosporine A present in the formulations of Examples 6-8 through the skin. Each formulation (25-35 mg) was tested in 3 different cells using pig ear skin (declared fit for human consumption by the Official Veterinary Service of the Government of Navarra and provided by the slaughterhouse "La Protectora S.A.") dermatomized to a final thickness of 400 ± 30 μm. For performance of the study a piece of porcine skin was placed on a cell an in contact with a fluid (PBS:EtOH 60:40) in a receptor chamber. The formulation to be evaluated was placed on the surface of the skin and the system was maintained under stirring at 32 ± 1 °C throughout the desired time period (of up to 24 hours). During this period, several receptor chamber samples (1 mL) were extracted (at 0h, 1 h, and 6 h), replacing the extracted volume with fresh fluid. Once the study was finished (at 24 h), the entire volume of the cell (7 mL) was collected and the skin was washed with the adequate medium to remove any non-absorbed excess product. This wash fluid was then collected and the skin preserved. Subsequently, all the fluids (wash and receptor chamber) together with the skin samples obtained in the study were kept as -20 °C in a freezer until their analysis.

*Formulation processing*

Each formulation was analyzed in triplicate. For the analysis of the formulation of Examples 6 and 7, 100 mg of said formulations were accurately weighed and filled to the mark with acetonitrile (ACN, Merck, HPLC grade) in a 5 mL volumetric flask. For the analysis of the formulation of Example 8, 100 mg of said formulation was accurately weighed and filled to the mark with acetonitrile (Merck, HPLC grade) in a 10 mL volumetric flask. All the samples were filtered through 0.22 μm membranes and injected onto the HPLC.

*Skin preparation*

Pig ears were obtained from the municipal slaughterhouse form animals slaughtered on the same day of the study. After reception, the ears were cleaned with water, their hair shaved carefully with a razor and finally biopsied and cleared of any adhering subcutaneous tissues with a scalpel. Then, the skin was dermatomized to a final thickness of 400 ± 30 μm with a dermatome (Braun, Acculan 3Ti). Once dermatomized, a visual inspection of the skin was performed to establish whether they had suffered any damage during the process. Only skin fragments that passed the macroscopical evaluation were used for the study. A specific micrometer was then used to measure the thickness of each skin and only fragments with thickness between 370 μm and 430 μm were accepted for the present study. Prior to the application of the formulations of Examples 6-8, the integrity of the skin barrier and the water tightness of the experimental model were verified by measuring the Trans Epidermal Water Loss (TEWL) for each diffusion cell. The measurement was performed directly on the donor compartment using an evaporimeter (Tewameter, Microcaya, TM300).

*Percutaneous absorption study*

The study was performed using Automatic Franz Diffusion Cells (Hanson Corporate). The cells were cleaned and conditioned with the receptor compartment fluid (phosphate buffer solution:ethanol (PBS:EtOH) 60:40) before the beginning of the study. Each dermatomized pig ear skin was mounted in horizontal position between the two parts of the cell demarcating two compartments, one on each side of the skin:

- receptor compartment: fluid applied to the lower side of the skin, consisting of 7 mL of PBS:EtOH (60:40), with a sampling port for sample collection, and

- donor compartment: Teflon cylinder (dosage wafer) with an accurately defined surface of 1.767 $cm^2$ applied to the upper side of the skin.

Each cell had a water jacket that allowed keeping the system at a constant temperature of 32 ± 1 °C at all times. Inside each cell there was a small magnet and a helix, set up at a constant stirring speed of 400 rpm to homogenize the fluid in the receptor compartment. Half an hour after the skin was mounted on the cell and conditioned, the integrity of the skin barrier and the water tightness of the experimental model were verified for each diffusion cell before the application of the formulations of Examples 6-8, by measurement of the TEWL. The measurement was performed directly on the donor compartment using an evaporimeter.
Finally, the corresponding formulation of Examples 6-8 was administered on each cell (between 22 and 34 mg). After administration, the surface of the skin was left open to the atmosphere of the laboratory in a non-occluded situation.
One milliliter samples of the receptor compartment were collected at the initial time point (0 h) and after 1 h and 6 h. The entire volume of the cell (7 mL) was collected after 24 h. At each sampling time, the withdrawn volume was replaced with fresh fluid (PBS:EtOH 60:40).
After 24 h and complete collection of the receptor compartment fluid, the remaining active ingredient on the surface of the skin was removed by washing the skin. The washing was performed by swiping with cotton buds. The cotton buds were placed in a Falcon tube and left open to dry for 15 h and then frozen at -20 °C until analyzed.
After washing the skin, the cells were dismantled and the skin was collected, placed in a Falcon tube and frozen until analyzed.

*HPLC method for detection of cyclosporine A in receptor chamber fluid*

The samples were analyzed by ultra high performance liquid chromatography in a Acquity UPLC equipment (Waters) using a UPLC column (BEH $C_{18}$, 1.7 μm, 50 x 2.1 mm, Waters) at 40 °C. To 50 μL of sample,[$^2H_{12}$]-cyclosporine A (Alsachim) was added (100 μL of 250 ng/mL of [$^2H_{12}$]-cyclosporine A to each sample) as internal standard and 350 μL methanol:water (8:2). 10 μL of the resulting solutions were injected.
An internal standard stock solution of [$^2H_{12}$]-cyclosporine A was prepared at a concentration of 1 mg/mL in DMSO. Then, 10 μL of this stock solution were added to 990 μL of methanol:$H_2O$ 1:1. Finally, 250 μL of the latter solution were added to 9750 μL of methanol:$H_2O$ 8:2 to give a solution of 250 ng/mL of [$^2H_{12}$]-cyclosporine A.
Mobile phase A was 2 mM ammonium acetate with 0.1% formic acid in water. Mobile phase B was 0.1% formic acid in methanol. The flow rate was 0.25 mL/min. The mobile phase gradient was as follows:

- initial: 20% A, 80% B
- 0.6 min: 20%A, 80% B
- 2.0 min: 5% A, 95% B
- 3.0 min: 20% A, 80% B

Detection was performed by tandem mass spectrometry (electrospray in positive mode) (TQD mass spectrometry system, Waters) as follows:

- cyclosporine A:

  MRM parameters: 1220.69 → 1203.70
  cone voltage: 40 V
  collision energy: 20 eV

- [$^2H_{12}$]-cyclosporine A:

  MRM parameters: 1232.70 → 1215.80
  cone voltage: 40 V
  collision energy: 20 eV

*HPLC method for detection of cyclosporine A in pig ear skin*

The samples were analyzed by ultra high performance liquid chromatography in a Acquity UPLC equipment (Waters) using a UPLC column (BEH $C_{18}$, 1.7 μm, 50 x 2.1 mm, Waters) at 40 °C.
The samples were extracted by addition of 10 mL of methanol to the skin sample and leave them mixing for

16-18 h. Then, the samples were sonicated for 1 h. The samples were then diluted before sample processing 1:20 with methanol:water (1:1) (50 µL of sample and 950 µL of methanol:water 1:1). To 100 µL of said extract sample, [$^2H_{12}$]-cyclosporine A (Alsachim) was added (100 µL of 500 ng/mL of [$^2H_{12}$]-cyclosporine A to each sample) as internal standard and 300 µL methanol:water (8:2). 10 µL of the resulting solutions were injected. Mobile phase A was 2 mM ammonium acetate with 0.1% formic acid in water. Mobile phase B was 0.1% formic acid in methanol. The flow rate was 0.25 mL/min. The mobile phase gradient was as follows:

- initial: 20% A, 80% B
- 0.6 min: 20%A, 80% B
- 2.0 min: 5% A, 95% B
- 3.0 min: 20% A, 80% B

Detection was performed by tandem mass spectrometry (electrospray in positive mode) (TQD mass spectrometry system, Waters) as follows:

- cyclosporine A:

  MRM parameters: 1220.69 → 1203.70
  cone voltage: 40 V
  collision energy: 20 eV

- [$^2H_{12}$]-cyclosporine A:

  MRM parameters: 1232.70 → 1215.80
  cone voltage: 40 V
  collision energy: 20 eV

*Calibration curve for detection of cyclosporine A in receptor chamber fluid*

A standard stock solution of cyclosporine A was prepared at a concentration of 1 mg/mL in DMSO. Then, the corresponding amount of this stock solution was added to the corresponding amount of methanol:$H_2O$ 1:1 to obtain the corresponding cyclosporine A calibration solutions at concentrations in the range from 5 to 1000 ng/mL.

These solutions were analyzed by ultra high performance liquid chromatography in a Acquity UPLC equipment (Waters) using a UPLC column (BEH $C_{18}$, 1.7 µm, 50 x 2.1 mm, Waters) at 40 °C. To 10 µL of cyclosporine A calibration solutions was added 990 µL of ethanol:phosphate buffer 0.01 M (4:6).

The calibration curve parameters (slope, intercept and correlation coefficient) were obtained by using the least squares calculation methods from cyclosporine A solutions at different concentrations.

*Calibration curve for detection of cyclosporine A in pig ear skin*

A standard stock solution of cyclosporine A was prepared at a concentration of 1 mg/mL in DMSO. Then, the corresponding amount of this stock solution was added to the corresponding amount of methanol:$H_2O$ 1:1 to obtain the corresponding cyclosporine A calibration solutions at concentrations in the range from 5 to 1000 ng/mL.

A blank matrix solution was prepared by dilution 1:20 of digested blank skin with methanol:$H_2O$ (1:1).The digested blank skin was prepared by addition of 10 mL of methanol to a skin sample that had been subjected to the Franz Cell Study without addition of any formulation, and then left mixing for 16-18 h. Then, the samples were sonicated for 1 h.

These solutions were analyzed by ultra high performance liquid chromatography in a Acquity UPLC equipment (Waters) using a UPLC column (BEH $C_{18}$, 1.7 µm, 50 x 2.1 mm, Waters) at 40 °C. To 10 µL of cyclosporine A calibration solutions 990 µL of blank matrix was added.

The calibration curve parameters (slope, intercept and correlation coefficient) were obtained by using the least squares calculation methods from cyclosporine A solutions at different concentrations.

*Formulation quantification*

The exact concentration of cyclosporine A (CyA) in each formulation was calculated against a calibration curve. The results are shown in the table below (SD is the standard deviation).

| *Formulation* | *Replicate* | *Signal (mAU)* | *CyA (%)* | *Average (%)* | *SD* |
|---|---|---|---|---|---|
| Example 6 | 1 | 12831.34 | 2.00 | 2.01 | 0.01 |
| | 2 | 11289.69 | 2.01 | | |
| | 3 | 11147.48 | 2.02 | | |
| Example 7 | 1 | 12187.23 | 2.02 | 2.02 | 0.02 |
| | 2 | 12758.40 | 2.00 | | |
| | 3 | 11248.83 | 2.04 | | |
| Example 8 | 1 | 15590.13 | 4.99 | 4.96 | 0.02 |
| | 2 | 13957.65 | 4.96 | | |
| | 3 | 14192.25 | 4.95 | | |

The receptor compartment results are provided in the tables below.

| *Formulation* | *Number of cells analyzed* | *Time (h)* | *Average accumulated CyA released (μg)* | Average accumulated CyA released *(μg/cm²)* |
|---|---|---|---|---|
| Example 6 | 3 | -1 | 0.000 ± 0.000 | 0.000 ± 0.000 |
| | 3 | 0 | 0.000 ± 0.000 | 0.000 ± 0.000 |
| | 3 | 1 | 0.000 ± 0.000 | 0.000 ± 0.000 |
| | 3 | 6 | 0.120 ± 0.207 | 0.068 ± 0.117 |
| | 3 | 24 | 0.309 ± 0.422 | 0.175 ± 0.239 |
| Example 7 | 3 | -1 | 0.000 ± 0.000 | 0.000 ± 0.000 |
| | 3 | 0 | 0.000 ± 0.000 | 0.000 ± 0.000 |
| | 3 | 1 | 0.000 ± 0.000 | 0.000 ± 0.000 |
| | 3 | 6 | 0.000 ± 0.000 | 0.000 ± 0.000 |
| | 3 | 24 | 0.092 ± 0.026 | 0.052 ± 0.014 |
| Example 8 | 3 | -1 | 0.000 ± 0.000 | 0.000 ± 0.000 |
| | 3 | 0 | 0.000 ± 0.000 | 0.000 ± 0.000 |
| | 3 | 1 | 0.000 ± 0.000 | 0.000 ± 0.000 |
| | 3 | 6 | 0.198 ± 0.098 | 0.112 ± 0.056 |
| | 3 | 24 | 0.662 ± 0.374 | 0.375 ± 0.212 |

| *Formulation* | *Number of cells analyzed* | *Average CyA in skin after 24 h (μg)* | *AverageCyAin skin after 24 h (μg /cm²)* |
|---|---|---|---|
| Example 6 | 3 | 5.521 ± 3.064 | 3.124 ± 1.734 |
| Example 7 | 3 | 13.715 ± 19.478 | 7.762 ± 11.023 |
| Example 8 | 3 | 44.099 ± 64.080 | 24.957 ± 36.265 |

The results show that formulations of Examples 6-8 are suitable for topical delivery of cyclosporine A.

## 6. Biological activity studies

The biological activity of formulations of Examples 6-8, applied topically, was assessed on the inflammation reaction produced by the induction of an allergic contact dermatitis on farm pigs' skin (a validated model of allergic dermatitis).As reference formulation a 0.1% tacrolimus commercially available formulation (Protopic 0.1%) was used.

*Test animals*

6 female (not siblings) pigs (Landrance x Large White from Prolabor) having a mean body weight of 11.0 ± 0.84 kg were used. The animals were kept under standard laboratory conditions and received standard porcine feed and tap water *ad libitum.*

*Administration route and volume*

The treatments were applied topically to circular areas approximately 2 cm in diameter (3.14 $cm^2$ surface), on the back of the animals. The amount of the test formulation to be applied for each treatment was 630 mg on each application site or the equivalent in volume.

*Activity on allergic contact dermatitis*

After an acclimatization period of nine days, the sensitization started on what was considered day 1 of the study by means of topical administration of 100 μL of 10% difluoronitrobenzene (DNFB, Sigma Aldrich) on the ears (medial aspects) and groins of the animals. On day 4 of the study, 100 μL of DNFB was administered at a concentration of 2% on the ears of each animal, avoiding the application site of day 1. The application of DNFB on days 1 and 4 was performed by spreading the formulation on the cited areas. Four days before the challenge test, the animals were shaved to assure the correct application of the treatments. On day 12 of the study, the challenge was induced by means of the topical application of 20μL of 1% DNFB on six application sites (2 cm in diameter) on each side of the back of the animals (12 application sites in total). The application sites were located in a dorsal position, avoiding the flexor area of the neck of the animals. The first application sites, with respect to a craniocaudal position, were reserved for the control (no treatment). The rest of treatments (including the treatment with the reference formulation) were applied after the control treatment position. Both the reference formulation treatment and the treatments corresponding to the test formulations were applied randomly in the application sites of all the animals. The six animals of the study were treated with the reference formulation and the formulations of Examples 6-8. The assigned treatments were applied on the aforementioned sensitized areas 0.5 and 6 hours after the induction of the challenge. The applications were performed in two phases, first, a small amount was applied and massaged until it was absorbed, and then the rest of the amount to be administered was applied to the same area. Before the application of the treatments at 6 h, the remainder of the test formulations was removed with gauze moistened with physiological saline. Each treatment was administered on two of the application sites on each animal (one application on each side of the back of the animal). Only 20 μL of 1% DNFB was applied to the control sites. Twenty-four hours after the induction of the challenge, the remainder of the test formulations was removed with a gauze moistened with physiological saline and approximately four minutes later, the areas of the skin where the treatments were applied were evaluated.

*Evaluation of results*

The intensity and extension of the erythema and the consistency of the lesions was assessed on a scale of 0-4 according to the following criteria:

| *Score* | *Intensity* | *Extension* | *Consistency* |
|---|---|---|---|
| 0 | No erythema | No erythema | Nomal findings |
| 1 | Barely perceptible | Macules of pinhead size | Nodules of pinhead size |
| 2 | Slight | Lentil-sized macules | Doughy, lentil-sized nodules |
| 3 | Moderate | Confluent macules | Confluent firm nodules |
| 4 | Severe | Diffuse | Diffuse hard lesions |

The means of the scores obtained were calculated for the different parameters to be evaluated, both per animal

and per treatment group. The mean, standard error (SE), and the standard error of the mean (SEM) were calculated for each treatment group. The percentage of inhibition was calculated for each treatment group using the following formula:

$$\%Inhibition = \frac{(Mean\ global\ score)_{control} - (Mean\ global\ score)_{treatment}}{(Mean\ global\ score)_{control}} x100$$

The values obtained for each animal were only accepted for the test when the score for the consistency lesion induced in the control treatment application site was greater than or equal to 2.

The criteria for the classification of the activity against the inflammation induced by the application of DNFB were the following:

| *Percentage of inhibition* | *Evaluation* |
|---|---|
| < 10% | Very low activity on allergic contact dermatitis |
| 10-40% | Slight activity on allergic contact dermatitis |
| 41-70% | Good activity on allergic contact dermatitis |
| 71-100% | Very good activity on allergic contact dermatitis |

*Results*

The percentage of inhibition of the different treatments (formulations of Examples 6-8 and the reference treatment with Protopic 0.1%) with respect to the control treatment are gathered in the table below:

| *Treatment* | *Intensity (%)* | *Extension (%)* | *Consistency (%)* | *Inhibition (%)* |
|---|---|---|---|---|
| Formulation of Example 6 | 60 | 65 | 69 | 64 |
| Formulation of Example 7 | 57 | 62 | 59 | 59 |
| Formulation of Example 8 | 60 | 68 | 66 | 64 |
| Protopic 0.1% | 60 | 68 | 69 | 65 |
| Control | 0 | 0 | 0 | 0 |

The formulations of Examples 6-8 showed good inhibitory response against the reaction induced by the application of DNFB.

## Claims

**1.** A topical pharmaceutical microemulsion comprising:

(a) from 0.1%w/w to 10% w/w of cyclosporine A relative to the total weight of the microemulsion,
(b) a half $C_{1-4}$-alkylester of a poly(methyl vinyl ether-co-maleic anhydride) (PVM/MA) copolymer,
(c) a non-volatile organic solvent capable of solubilizing component (b)other than 2-(2-ethoxyethoxy)ethanol and surfactants having an HLB value from 10 to 18,
(d) a volatile alcohol,
(e) 2-(2-ethoxyethoxy)ethanol,
(f) a medium chain triglyceride,
(g) a surfactant or surfactant mixture having an HLB value from 10 to 18,
(h) optionally water, and
(i) optionally triacetin.

**2.** The microemulsion according to claim 1, wherein component (b) is the half n-butyl ester of a PVM/MA copolymer.

**3.** The microemulsion according to any one of the preceding claims, wherein component (c) is propylene glycol.

4. The microemulsion according to any one of the preceding claims, wherein component (d) is ethanol.

5. The microemulsion according to any one of the preceding claims, wherein component (f) is caprylic/capric acid triglyceride.

6. The microemulsion according to any one of the preceding claims, wherein component (g) is either polysorbate 80 or a mixture of polysorbate 80 and polysorbate 20.

7. The microemulsion according any one of the preceding claims, wherein component (a) is present in a concentration of 1% w/w to 7% w/w relative to the total weight of the microemulsion, preferably in a concentration of 1.5% w/w to 5% w/w, more preferably in a concentration of 2% w/w to 5% w/w.

8. The microemulsion according to any one of the preceding claims, comprising:

(a) from 1% w/w to 7% w/w of cyclosporine A,
(b)from 0.02% w/w to 5% w/w of a half $C_{1-4}$-alkylester of a poly(methyl vinyl ether-co-maleic anhydride) (PVM/MA) copolymer,
(c)from 5% w/w to 15% w/w of a non-volatile organic solvent capable of solubilizing component (b)other than 2-(2-ethoxyethoxy)ethanol and surfactants having an HLB value from 10 to 18 and triacetin,
(d)from 0.05% w/w to 15% w/w of a volatile alcohol,
(e) from 20% w/w to 25% w/w of 2-(2-ethoxyethoxy)ethanol,
(f) from 2% w/w to 30% w/w of a medium chain triglyceride,
(g)from 10% w/w to 50% w/w of a surfactant or surfactant mixture having an HLB value from 10 to 18,
(h) optionally from 15% w/w to 25% w/w of water, and
(i) optionally from 10% w/w to 20% w/w of triacetin,

wherein w/w is the weight of each component relative to the total weight of the microemulsion.

9. The microemulsion according to claim 8, comprising:

(a) from 1% w/w to 7% w/w of cyclosporine A,
(b)from 0.02% w/w to 5% w/w of a half $C_{1-4}$-alkylester of a poly(methyl vinyl ether-co-maleic anhydride) (PVM/MA) copolymer,
(c)from 5% w/w to 15% w/w of a non-volatile organic solvent capable of solubilizing component (b)other than 2-(2-ethoxyethoxy)ethanol and surfactants having an HLB value from 10 to 18 and triacetin,
(d)from 5% w/w to 15% w/w of a volatile alcohol,
(e) from 20% w/w to 25% w/w of 2-(2-ethoxyethoxy)ethanol,
(f) from 10% w/w to 30% w/w of a medium chain triglyceride,
(g)from 10% w/w to 20% w/w of a surfactant or surfactant mixture having an HLB value from 10 to 18, and
(j) from 10% w/w to 20% w/w of triacetin,

wherein w/w is the weight of each component relative to the total weight of the microemulsion.

10. The microemulsion according to any one of claims 1 to 8, comprising:

(a) from 1% w/w to 7% w/w of cyclosporine A,
(b)from 0.02% w/w to 5% w/w of a half $C_{1-4}$-alkylester of a poly(methyl vinyl ether-co-maleic anhydride) (PVM/MA) copolymer,
(c)from 5% w/w to 15% w/w of a non-volatile organic solvent capable of solubilizing component (b)other than 2-(2-ethoxyethoxy)ethanol and surfactants having an HLB value from 10 to 18,
(d)from 1% w/w to 10% w/w of a volatile alcohol,
(e) from 20% w/w to 25% w/w of 2-(2-ethoxyethoxy)ethanol,
(f) from 2% w/w to 10% w/w of a medium chain triglyceride,
(g)from 30% w/w to 50% w/w of a surfactant or surfactant mixture having an HLB value from 10 to 18, and
(h)from 15% w/w to 25% w/w of water,

wherein w/w is the weight of each component relative to the total weight of the microemulsion.

**11.** The microemulsion according to claim 10, comprising:

(a) from 1% w/w to 5% w/w of cyclosporine A,
(b)from 0.02% w/w to 5% w/w of the half n-butyl ester of a PVM/MA copolymer,
(c) from 5% w/w to 10% w/w of propylene glycol,
(d)from 1% w/w to 6% w/w of a ethanol,
(e) from 20% w/w to 25% w/w of 2-(2-ethoxyethoxy)ethanol,
(f) from 2% w/w to 8% w/w of a caprylic/capric acid triglyceride,
(g)from 20% w/w to 30% w/w of polysorbate 80 and from 10% w/w to 20% w/w of polysorbate 20, and
(h)from 15% w/w to 25% w/w of water,

wherein w/w is the weight of each component relative to the total weight of the microemulsion.

**12.** The microemulsion according to any one of the preceding claims, which is devoid of oleic acid and/or additional preservatives.

**13.** A process for producing a topical pharmaceutical microemulsion as defined in any one of claims 1 to 12 comprising:

(i) preparing an homogeneous mixture comprising cyclosporine A and the medium chain triglyceride,
(ii) preparing an homogeneous solution comprising the half $C_{1-4}$-alkylester of a poly(methyl vinyl ether-co-maleic anhydride) (PVM/MA) copolymer, the volatile alcohol and the non-volatile organic solvent other than 2-(2-ethoxyethoxy)ethanol and surfactants having an HLB value from 10 to 18;
(iii) adding the solution obtained in step (ii) to the mixture obtained in step (i) under stirring,
(iv) adding 2-(2-ethoxyethoxy)ethanol, the surfactant or surfactant mixture having an HLB value from 10 to 18, and optionally triacetin to the mixture obtained in step (iii) and stirring until a homogeneous solution is obtained, and
(v) optionally adding water to the mixture obtained in step (iv) and stirring until a homogeneous solution is obtained.

**14.** A topical pharmaceutical microemulsion as defined in any one of claims 1 to 12 for use as a human or veterinary medicament.

**15.** A topical pharmaceutical microemulsion as defined in any one of claims 1 to 12 for use in the prevention and/or treatment of a disease selected from the group consisting of psoriasis, atopic dermatitis, allergic dermatitis, pyoderma gangrenosum, refractory chronic idiopathic urticaria, dyshidrotic eczema, Behçet disease, pityriasis rubra pilaris, dermatomyositis, pemphigus vulgaris, benign familiar pemphigus, pemphigus foliaceus and erythematosus, epidermolysis bullosa acquisita, photodermatoses, lichen planus, prurigonodularis, alopecia areata, eosinophilic pustular folliculitis, granulomatous folliculitis and furunculosis, mular folliculitis, hidradenitis suppurativa, scleroderma, vitiligo, eosinophilic granuloma complex, perianal fistulas, sebaceous adenitis, juvenile cellulitis, vesicular cutaneous lupus erythematosus, erythema multiforme, discoid lupus erythematosus, sterile nodular panniculitis, metatarsal fistulae, nasal arteritis, ulcerative dermatosis of nasal philtrum, facial dermatitis, sterile granuloma or pyogranuloma syndrome, pseudopelade, cutaneous reactive histiocytosis, feline plasma cell pododermatitis, vasculitis and ischemic dermatopathy; preferably psoriasis, atopic dermatitis or allergic dermatitis.

Europäisches Patentamt
European Patent Office
Office européen des brevets

# EUROPEAN SEARCH REPORT

Application Number
EP 16 38 2001

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2014/189251 A1 (TAE JOON PHARM CO LTD) 27 November 2014 (2014-11-27)<br>* the whole document *<br>----- | 1-15 | INV.<br>A61K9/107<br>A61K38/13 |
| Y | US 5 962 017 A (HAUER BIRGIT [DE] ET AL) 5 October 1999 (1999-10-05)<br>* claims 1-25 *<br>* examples 1-7 *<br>----- | 1-15 | |
| Y | WO 01/28520 A1 (VESIFACT AG [CH]; SUPERSAXO ANDREAS WERNER [CH]; WEDER MARC ANTOINE [C) 26 April 2001 (2001-04-26)<br>* claims 1-16 *<br>* examples 1,2 *<br>----- | 1-15 | |
| Y | US 2007/015691 A1 (CHANG JAMES N [US] ET AL) 18 January 2007 (2007-01-18)<br>* claim 1 *<br>* paragraph [0873] *<br>----- | 1-15 | |

TECHNICAL FIELDS SEARCHED (IPC)

A61K

The present search report has been drawn up for all claims

| Place of search | Date of completion of the search | Examiner |
|---|---|---|
| Munich | 28 April 2016 | Sindel, Ulrike |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 16 38 2001

This annex lists the patent family members relating to the patent documents cited in the above-mentioned European search report.
The members are as contained in the European Patent Office EDP file on
The European Patent Office is in no way liable for these particulars which are merely given for the purpose of information.

28-04-2016

| Patent document cited in search report | Publication date | Patent family member(s) | Publication date |
|---|---|---|---|
| WO 2014189251 A1 | 27-11-2014 | NONE | |
| US 5962017 A | 05-10-1999 | AT 403435 B | 25-02-1998 |
| | | AU 627220 B2 | 20-08-1992 |
| | | AU 4140089 A | 22-03-1990 |
| | | BE 1003105 A5 | 26-11-1991 |
| | | BG 60525 B2 | 28-07-1995 |
| | | CA 1332150 C | 27-09-1994 |
| | | CH 679118 A5 | 31-12-1991 |
| | | DE 3930928 A1 | 22-03-1990 |
| | | DK 171433 B1 | 28-10-1996 |
| | | ES 2020738 A6 | 16-09-1991 |
| | | FI 894342 A | 17-03-1990 |
| | | FR 2636534 A1 | 23-03-1990 |
| | | GB 2222770 A | 21-03-1990 |
| | | GR 890100583 A | 31-10-1990 |
| | | HK 86593 A | 27-08-1993 |
| | | HU 211685 A9 | 28-12-1995 |
| | | ID 17863 A | 29-01-1998 |
| | | IE 60764 B1 | 10-08-1994 |
| | | IL 91642 A | 12-04-1994 |
| | | IT 1232243 B | 28-01-1992 |
| | | JP H0725690 B2 | 22-03-1995 |
| | | JP H02121929 A | 09-05-1990 |
| | | KR 20070108289 A | 09-11-2007 |
| | | LU 87586 A1 | 07-05-1991 |
| | | LV 5749 A4 | 20-12-1996 |
| | | NL 8902315 A | 17-04-1990 |
| | | NO 893678 A | 19-03-1990 |
| | | NZ 230660 A | 25-06-1992 |
| | | PH 31059 A | 05-02-1998 |
| | | PT 91731 A | 30-03-1990 |
| | | SE 514303 C2 | 05-02-2001 |
| | | SE 8903042 L | 11-05-1990 |
| | | SG 50793 G | 25-06-1993 |
| | | US 5741512 A | 21-04-1998 |
| | | US 5866159 A | 02-02-1999 |
| | | US 5916589 A | 29-06-1999 |
| | | US 5962014 A | 05-10-1999 |
| | | US 5962017 A | 05-10-1999 |
| | | US 6024978 A | 15-02-2000 |
| | | US 2003143250 A1 | 31-07-2003 |
| WO 0128520 A1 | 26-04-2001 | AT 333267 T | 15-08-2006 |
| | | AU 7638200 A | 30-04-2001 |
| | | AU 7638300 A | 30-04-2001 |

EPO FORM P0459

For more details about this annex : see Official Journal of the European Patent Office, No. 12/82

page 1 of 2

## ANNEX TO THE EUROPEAN SEARCH REPORT ON EUROPEAN PATENT APPLICATION NO. EP 16 38 2001

This annex lists the patent family members relating to the patent documents cited in the above-mentioned European search report.
The members are as contained in the European Patent Office EDP file on
The European Patent Office is in no way liable for these particulars which are merely given for the purpose of information.

28-04-2016

| Patent document cited in search report | Publication date | Patent family member(s) | Publication date |
|---|---|---|---|
| | | AU 7767600 A | 30-04-2001 |
| | | DK 1289502 T3 | 13-11-2006 |
| | | EP 1289502 A1 | 12-03-2003 |
| | | EP 1408930 A1 | 21-04-2004 |
| | | ES 2267570 T3 | 16-03-2007 |
| | | JP 4913301 B2 | 11-04-2012 |
| | | JP 2003512312 A | 02-04-2003 |
| | | US 8158134 B1 | 17-04-2012 |
| | | WO 0128518 A1 | 26-04-2001 |
| | | WO 0128519 A1 | 26-04-2001 |
| | | WO 0128520 A1 | 26-04-2001 |
| US 2007015691 A1 | 18-01-2007 | US 2007015691 A1 | 18-01-2007 |
| | | US 2012270805 A1 | 25-10-2012 |
| | | US 2014045772 A1 | 13-02-2014 |
| | | US 2015343017 A1 | 03-12-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 8642556 B2
- US 8629111 B2
- US 8618064 B2
- US 8633162 B2
- US 8648048 B2
- US 8685930 B2
- US 5891846 A


### Non-patent literature cited in the description


- **AMOR et al.** *J. Am. Acad. Dermatol.,* 2010, vol. 63, 925-946
- **KOVALIK et al.** *The Veterinary Journal,* 2012, vol. 193
- **PALMEIRO.** *Vet. Clin. Small Anim.,* 2013, vol. 43, 154-171
- **CARIDI et al.** *Topical Calcineurin Inhibitors in the Treatment of Vitiligo in Bitiligo - Management and Therapy,* 2001
- **GODDERERIS C. et al.** *International Journal of Pharmaceutics,* 2006, vol. 312, 187-195
- **GRIFFIN.** *Journal of the Society of Cosmetic Chemists,* 1949, vol. 1, 311-326
- Handbook of pharmaceutical excipients. Pharmaceutical Press and the American Pharmacists Association, 2009